# EUROPEAN PATENT APPLICATION

(11) **EP 2 368 552 A1**
(43) Date of publication of application: **28.09.2011**
(21) Application number: 10157762.5
(22) Date of filing: 25.03.2010
(51) Int. Cl.: A61K 31/4535, A61K 31/522, A61K 31/70, A61P 3/10

(54) **1-[(3-cyano-pyridin-2-yl)methyl]-3-methyl-7-(2-butyn-1-yl)-8-[3-(r)-amino-piperidin-1-yl]-xanthine for the treatment of a metabolic disorder of a predominantly carnivorous non-human animal**

(71) Applicant: Boehringer Ingelheim Vetmedica GmbH, 55216 Ingelheim am Rhein (DE)
(72) Inventor: The designation of the inventor has not yet been filed
(74) Representative: Hammann, Heinz

(57) **Abstract**

The present invention provides a pharmaceutical composition comprising 1-[(3-cyano-pyridin-2-yl)methyl]-3-methyl-7-(2-butyn-1-yl)-8-[3-(R)-amino-piperidin-1-yl]-xanthine or a pharmaceutically acceptable form thereof for the therapy of a metabolic disorder or metabolic disease of a predominantly carnivorous non-human animal. It is especially useful for the therapy of diabetes and related diseases of predominantly carnivorous mammals like cats or dogs. It further provides respective uses of such compositions and of 1-[(3-cyano-pyridin-2-yl)methyl]-3-methyl-7-(2-butyn-1-yl)-8-[3-(R)-amino-piperidin-1-yl]-xanthine or pharmaceutically acceptable forms thereof.

## Description

### FIELD OF THE INVENTION

The present invention relates to veterinary medicine, esp. to the treatment of metabolic disorders of predominantly carnivorous non-human animals.

### BACKGROUND OF THE INVENTION

Animals, especially mammals are affected by metabolic disorders. Especially for predominantly carnivorous mammals like dogs and cats, a number of metabolic disorders are known, like ketoacidosis, pre-diabetes, diabetes mellitus type 1 or type 2, insulin resistance, obesity, hyperglycemia, hyperinsulinemia, elevated blood levels of fatty acids and/or of glycerol, Syndrome X, atherosclerosis, inflammation of the pancreas and/or inflammation of adipose tissue. Several of these disorders are correlated with each other. Among these disorders, diabetes mellitus type II is gaining more and more importance which can at least partially be ascribed to changing living and feeding behaviour of companion animals during the last years.

Diabetes mellitus is characterized by disturbances in carbohydrate, protein and triglyceride metabolism based on a relative or absolute lack of insulin. It is a relatively common endocrinopathy in carnivorous animals like the cat. The incidence for cat is approximately 0.5%. Several risk factors have been identified: age, obesity, neutering and gender. Male, castrated, obese and old (>10 years) cats have probably the greatest risk to develop diabetes mellitus.

The current classification divides diabetes mellitus into three classes:
(1.) Type 1 which results from the loss of function of insulin secreting cells, e.g. by immunologic destruction of beta cells or insulin auto-antibodies (juvenile diabetes);
(2.) Type 2 which results from a failure of the insulin stimulated cells to respond properly to insulin stimuli; it can also be cause e.g. by amyloid accumulation in in β-cells; type 2 usually develops during a long time of the so called pre-diabetes state;
(3.) secondary diabetes mellitus which can due to diabetogenic drugs (e.g. long-acting glucosteroids, megestrol acetat, etc.) or to other primary diseases like pancreatitis, pancreas adenocarcinoma, cushing, hypo- or hyperthyroidism, growth-hormone produing tumors resuling in acromegaly.

Diabetes mellitus type 2 is a growing problem around the developed world, especially for cat populations. The lifestyle changes of cat owners are mirrored in their cats - increasingly they are kept indoors, with reduced activity levels, and fed a calorie-rich diet, leading to obesity and predisposition to diabetes mellitus Type 2. As these trends continue, the incidence of diabetes mellitus in cats is sure to rise accordingly.

The same tendency can be seen at other companion animals, including others that are predominantly carnivorous. E.g. for dogs, it has been shown that obesity leads to profound changes in glucose disposal and insulin secretion (Hoenig, M. (2002): Comparative aspects of diabetes mellitus in dogs and cats; Mol. Cell. Endocrin., 197, 221-229).

Several oral hypoglycaemic drugs are approved for the treatment of type 2 diabetes mellitus in humans. These drugs work by stimulating pancreatic insulin secretion in a glucose-independent manner (sulfonylureas, meglitinides), stimulating pancreatic insulin secretion in a glucose-dependent manner (DPP IV inhibitors), by enhancing tissue sensitivity to insulin (biguanides, thiazolidinediones), or by slowing postprandial intestinal glucose absorption (alpha-glucosidase inhibitors). Oral medications like Glipizide (sulfonylurea) work in some small proportion of cats, but these drugs may be completely ineffective if the pancreas is not working. Worse, glipizide and other oral hypoglycaemic drugs have been shown in some studies to generate site effects such as vomiting and icterus and to damage the pancreas even further and by this reducing the chances of remission for cats. They have also been shown to cause liver damage.

Treatment with insulin is currently the gold standard to treat diabetes mellitus in predominantly carnivorous animals, especially dogs. Unlike for humans, for carnivorous mammals, esp. for cats, only one porcine insulin zinc preparation from the company Intervet/ScheringPlough is approved at the time being. However, only the UK has additional Insulin approved from Pfizer (Insuvet Neutral, Unsuvet Lente, Unsuvet Protamine Zinc). Cats are notoriously unpredictable in their response to exogenous insulin. No single type of insulin is routinely effective in maintaining control of glycemia, even with twice a day administration, in the field of companion animals.

Such insulin therapy is based upon insulin injection by the owner at home, ideally twice daily, along with dietary modification. Even with strict compliance from the owner control is often poor and secondary problems common - and many owners find it impossible to achieve such levels of compliance and synchronization of food intake and insulin injection is impossible in the majority of cases. Ultimately many cats with diabetes mellitus are euthanized because of the disease.

Consequently, a treatment that would allow better compliance and therefore better glycemic control would help to attenuate the progression of the disease and delay or prevent onset of complications in many animals.

Several oral anti-diabetics are described for the therapy of human diabetes. Among these, the substance group of xanthine derivatives should be mentioned. They are disclosed generically by WO 02/068420 A1 as inhibitors of Dipeptidyl-Peptidase IV (DPP IV). Among these, the certain compound BI-1356 (also named Linagliptin as listed in the WHO database of International Nonproprietary Names), has been further developed and is envisaged for the therapy of humans. This is supported by e.g. Rungby, J. (2009): Inhibition of dipeptidyl peptidase 4 by BI-1356, a new drug for the treatment of beta-cell failure in type 2 diabetes: Exp. Op. Invest. Drugs, 18(6), 835-838.

DPP IV-inhibitors have already been administered to animals, but only for test purposes. For example, the effects of the compound 1-[(3-Methyl-isochinoline-1-yl)methyl]-3-methyl-7-(2-butin-1-yl)-8-((R)-3-amino-piperidine-1-yl)-xanthine after an administration to rats and to cats, have been described by Sandel et al. (2006) in Tieräirztl. Prax., 34, 132-137. It has been shown that the effect of GLP-1 could be prolonged by this compound.

Another DPP IV-inhibitor, called NVP-DPP728 is described to reduce the plasma glucagon concentration in cats (Furrer et al.; to be published in The Veterinary Journal*).*

However, there are no oral anti-diabetics approved for veterinary medicine. This might be due to the observation that additionally to their desired ability to increase insulin in a glucose-independent manner, they also carry the risk of hypoglycaemia due to inadequate insulin secretion in animals. This problem is even worse for predominantly carnivorous mammals like dogs or cats because the energy metabolism of such animals is optimized for protein rich and carbohydrate low food and is thus not comparable to the human metabolism.

Effects and adverse effects of oral antidiabetics administered to cats are summarized in "Canine and feline endocrinology and reproduction, Third Edition, Feldmann, E.C. and Nelsen, R.W., publisher: Saunders / ISBN: 978-0-7216-9315-6), chaper 12: "Feline Diabetes mellitus", pages 539-579, table 12-4. This table as well as the accompanying text (pages 555-561) disclose that only 25% of cats respond to the treatment of sulfonylureas (like glipizide), which patients are still confronted with adverse effects like vomiting and hypoglycemia. An even worse value of efficacy is described for biguanides like metformin which is also accompanied by adverse effects like vomoting and weight loss. The other three compound groups disclosed there (meglitinides, thiazolidinediones and α-glucosidase inhibitors) are described to exert an even unknown efficacy.

Accordingly, there is a need for an improved treatment of metabolic diseases of non-human animals, esp. of diabetes type 2 of cats.

Advantageously, such an improved treatment is further comprised by one or more of the following features:
- the possibility for an oral treatment;
- the possibility for a subclinical treatment, e.g. the reduction of hyperglycaemia during the sublinical, pre-diabetes state;
- the possibility for the prevention and/or delay of the onset of diabetes mellitus;
- an improvement in glucose tolerance in glucose impaired patients;
- an enhancing effect for the long-term pancreatic β-cell function, advantageously by an increase in β-cell mass;
- an improvement in the safety/tolerability profile versus insulin, esp. a lowered risk of hypoglycaemia and/or no weight gain; and
- an anti-obesity effect.

### SUMMARY OF THE INVENTION

The present invention provides a pharmaceutical composition comprising 1-[(3-cyano-pyridin-2-yl)methyl]-3-methyl-7-(2-butyn-1-yl)-8-[3-(R)-amino-piperidin-1-yl]-xanthine or a pharmaceutically acceptable salt thereof for the treatment of a metabolic disorder or metabolic disease of a predominantly carnivorous non-human animal.

Without wishing to be bound by this theory it is believed in the context of this invention that the compound relevant for the invention acts in the signaling transduction pathway of the gut hormone GLP-1 which induces insulin. In mammals GLP-1 has got a short *in vivo* half-life of about 5 minutes, due to rapid degradation. The predominant proteolytic route occurs via cleavage of the 2 N-terminal amino acids by DPP IV. 1-[(3-cyano-pyridin-2-yl)methyl]-3-methyl-7-(2-butyn-1-yl)-8-[3-(R)-amino-piperidin-1-yl]-xanthine or any pharmaceutically acceptable form thereof is now believed to act as an inhibitor of dipeptidyl peptidase IV (DPP IV). In turn, the inhibition of DPP IV decreases the GLP-1 proteolysis and thus prolongs the half-life of endogenous full-length (active) GLP-1 and in doing so leads to increased plasma levels of glucagon like peptide 1 (GLP-1).

As a consequence, GLP-1 induces the secretion of insulin from pancreatic β-cells in a glucose dependent manner. A reduction of glucagon levels as well as an enhancement of long-term pancreatic β-cell function *in vivo* are potentially additional beneficial features of GLP-1 elevation, caused by 1-[(3-cyano-pyridin-2-yl)methyl]-3-methyl-7-(2-butyn-1-yl)-8-[3-(R)-amino-piperidin-1-yl]-xanthine or any pharmaceutically acceptable form thereof. Especially the enhancement of long-term pancreatic β-cell function can be characterized as a disease modifying effect; GLP-1 agonism preserves β-cell mass by increased proliferation and decreased apoptosis which effects can be characterized as a β-cell regeneration effect. Further effects of GLP-1 elevation according to the invention include slowing of gastric motility and induction of satiety.

This hypothesis is supported by experimental data: In vitro, 1-[(3-cyano-pyridin-2-yl)methyl]-3-methyl-7-(2-butyn-1-yl)-8-[3-(R)-amino-piperidin-1-yl]-xanthine, for example administered in the form of its monohydrochloride, inhibits DPP IV with an IC50 of about 1 nM using a dipeptide artificial substrate or about 0.18 nM using GLP-1 as one of the natural (and the most therapeutically relevant) substrates. Additional mechanistic studies indicated that this compound is a competitive and reversible inhibitor of DPP IV. Studies were also conducted to assess the inhibitory effect of this compound on plasma DPP IV activity in several species. 1-[(3-cyano-pyridin-2-yl)methyl]-3-methyl-7-(2-butyn-1-yl)-8-[3-(R)-amino-piperidin-1-yl]-xanthine inhibited plasma DPP IV activity in a concentration dependent manner with IC50s of about 1.7nM, 0.8nM, 1.3nM and 0.6 nM versus mouse, rat, dog and human plasma, respectively.

As can further be concluded from the experimental part of this application, 1-[(3-cyano-pyridin-2-yl)methyl]-3-methyl-7-(2-butyn-1-yl)-8-[3-(R)-amino-piperidin-1-yl]-xanthine efficaciously inhibits DPP IV activity in the plasma of different species including cats and improves glucose tolerance in normal and diabetic rodent models. In cat, also an increase of GLP-1 was demonstrated in normal cats challenged with glucose. Based on the results of the PK/PD profiling studies in can be concluded that the compound relevant for the invention is a long-acting compound which has the potential to sufficiently improve glycemic control especially in cats. It has been demonstrated that 1-[(3-cyano-pyridin-2-yl)methyl]-3-methyl-7-(2-butyn-1-yl)-8-[3-(R)-amino-piperidin-1-yl]-xanthine monohydrochloride leads to an increase of GLP-1 if challenged with glucose, meaning, a compound relevant for the invention has also the potential to have an effect on disease progression.

Besides the inhibitory effect of 1-[(3-cyano-pyridin-2-yl)methyl]-3-methyl-7-(2-butyn-1-yl)-8-[3-(R)-amino-piperidin-1-yl]-xanthine on DPP-IV, described above, it therefore lies also within the ambit of the application to enhance the activity of GLP-1 directly. Further included are other routes of enhancing the activity of GLP-1 which might not be revealed at the time being.

Especially for the treatment of predominantly carnivorous animals like dogs and esp. cats, 1-[(3-cyano-pyridin-2-yl)methyl]-3-methyl-7-(2-butyn-1-yl)-8-[3-(R)-amino-piperidin-1-yl]-xanthine shows better experimental results than other DPP IV inhibitors known from the state of the art.

Further data to support this general concept as well as specific uses and adavantages of this compound discussed intensively below, are presented in the experimental part of this application. Those data, however, are intended to highlight the invention with regard to specific effects without any limitation of the scope of the invention.

A second aspect provides for the use of a pharmaceutical composition according to the invention for the treatment of a metabolic disorder or metabolic disease of a predominantly carnivorous non-human animal.

A further aspect provides for the use of 1-[(3-cyano-pyridin-2-yl)methyl]-3-methyl-7-(2-butyn-1-yl)-8-[3-(R)-amino-piperidin-1-yl]-xanthine or a pharmaceutically acceptable salt thereof for the preparation of a pharmaceutical composition for the treatment of a metabolic disorder or metabolic disease of a predominantly carnivorous non-human animal.

A further aspect of the present invention pertains to the use of 1-[(3-cyano-pyridin-2-yl)methyl]-3-methyl-7-(2-butyn-1-yl)-8-[3-(R)-amino-piperidin-1-yl]-xanthine or a pharmaceutically acceptable salt thereof for the treatment of a metabolic disorder or metabolic disease of a non-human animal.

These and other aspects of the present invention are described herein by reference to the following figures and examples.

### DETAILED DESCRIPTION OF THE PRESENT INVENTION

All values and concentrations presented herein are subject to inherent variations acceptable in biological science within an error of ± 10%. The term "about" also refers to this acceptable variation.

Inhibitors of DPP IV belonging to the structural class of xanthine derivatives are disclosed generically by WO 02/068420 A1.

The compound 1-[(3-cyano-pyridin-2-yl)methyl]-3-methyl-7-(2-butyn-1-yl)-8-[3-(R)-amino-piperidin-1-yl]-xanthine is described explicitly in WO 2005/085246 A1, example 1 (52), its mono- and di-hydrochloride as well as polymorphs of the free base and the hydrochlorides are described in WO 2007/014886 A1. It can be described by the following chemical structure (I):

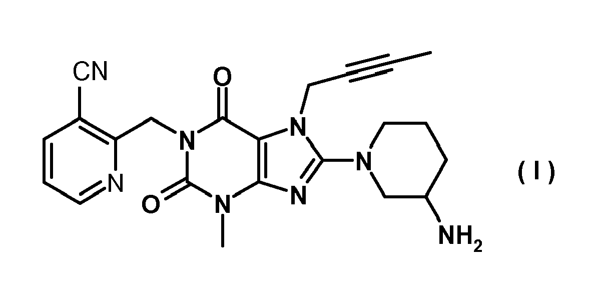

These applications disclose methods for the chemical synthesis of this compound along with its salts, hydrates and other chemical forms. Based on this information, the synthesis of related salts, prodrug esters, stereoisomers, solvates and of other chemical forms thereof is possible for a person skilled in the art. Alternative ways might be developed and respectively derived compounds are incorporated accordingly.

The present invention pertains to the compound 1-[(3-cyano-pyridin-2-yl)methyl]-3-methyl-7-(2-butyn-1-yl)-8-[3-(R)-amino-piperidin-1-yl]-xanthine in the form of the free base as well every other chemical form, including salts, prodrug esters, stereoisomers, and solvates, especially any pharmaceutically acceptable form thereof. All these forms are able to exert the above described physiological activities. Preferred is 1-[(3-cyano-pyridin-2-yl)methyl]-3-methyl-7-(2-butyn-1-yl)-8-[3-(R)-amino-piperidin-1-yl]-xanthine monohydrochloride, especially in crystalline form, preferentially in a polymorphic form as described in WO 2007/014886 A1. The examples disclosed in this application have been elaborated by use of the respective hydrochloride, i.e. with of 1-[(3-cyano-pyridin-2-yl)methyl]-3-methyl-7-(2-butyn-1-yl)-8-[3-(R)-amino-piperidin-1-yl]-xanthine monohydrochloride.

Throughout the present specification all of these forms are summarized under the name 1-[(3-cyano-pyridin-2-yl)methyl]-3-methyl-7-(2-butyn-1-yl)-8-[3-(R)-amino-piperidin-1-yl]-xanthine, also defined as "compound relevant for the invention".

Pharmaceutical compositions in the sense of the present invention are all kinds of chemical compositions comprising a pharmaceutically active compound of the present invention and excipients that support the intended medical effect. Such other excipients are known to a person skilled in the art. Useful excipients are for example antiadherents (used to reduce the adhesion between the powder (granules) and the punch faces and thus prevent sticking to tablet punches), binders (solution binders or dry binders that hold the ingredients together), coatings (to protect tablet ingredients from deterioration by moisture in the air and make large or unpleasant-tasting tablets easier to swallow), disintegrants (to allow the tablet to break upon dilution), fillers, diluents, flavours, colours, glidants (to promote powder flow by reducing interparticle friction and cohesion), lubricants (to prevent ingredients from clumping together and from sticking to the tablet punches or capsule filling machine), preservatives, sorbents sweeteners etc.

A treatment according to the present invention is a medical treatment or cure of a metabolic disorder and/or a metabolic disease. A disorder is understood to be a functional abnormality or disturbance of the normal body functions of an organism. A disease (= medical problem) is understood to be an abnormal condition of an organism that impairs bodily functions, associated with specific symptoms and signs. This includes any tendency of the body to develop a disease: For example, pre-diabetes might not always be evaluated as a disease itself. However, as it has got a tendency or at least a risk to develop further into diabetes and thus lies within the ambit of the present invention, which pertains to the treatment of clinical as well as (yet) sub-clinical metabolic disorders or metabolic diseases.

Metabolic disorders or metabolic diseases in the sense of the present invention are all kinds of disturbances of the energy metabolism, affecting e.g. the turnover of carbohydrates and/or of fat. It is preferred to affect the control of the energy metabolism, especially the glucose metabolism by influencing the responsible regulatory network, e.g. via modulation of the activity and/or concentrations of the hormones GLP-1 and/or insulin. The treatment of glucose metabolism disorders or diseases lies in the center of the underlying invention; such disorders or diseases are disorders of gluconeogenesis, hyperglycemia, ketosis (esp. ketoacidosis), diabetes mellitus (esp. diabetes mellitus type 2). Other disorders or diseases of the energy metabolism within the ambit of this invention are obesity, hyperglycemia, elevated blood levels of fatty acids and/or of glycerol, Syndrome X, atherosclerosis, inflammation of the pancreas and/or inflammation of adipose tissue. In the clinic such disturbances are correlated with each other which makes the invention a suitable approach for the therapy of several of these disorders or diseases as well as a means of prophylaxis of one or more of these disorders or diseases.

All living organims are characterized by energy metabolism. As far as they are non-human animals that feed predominantly on meat (i.e. that are not predominantly vegetarian), they are prone to medical treatments by pharmaceutical compositions according to the present invention.

In one embodiment the pharmaceutical composition according to the invention comprises the compound relevant for the invention (1-[(3-cyano-pyridin-2-yl)methyl]-3-methyl-7-(2-butyn-1-yl)-8-[3-(R)-amino-piperidin-1-yl]-xanthine) in the form of a salt. This is in a preferred embodiment 1-[(3-cyano-pyridin-2-yl)methyl]-3-methyl-7-(2-butyn-1-yl)-8-[3-(R)-amino-piperidin-1-yl]-xanthine monohydrochloride (= 1-[(3-cyano-pyridin-2-yl)methyl]-3-methyl-7-(2-butyn-1-yl)-8-[3-(R)-amino-piperidin-1-yl]-xanthine CL).

A preparation of this salt can also comprise additional molecules and/or ions of H₂O, HCI, H⁺ and/or Cl⁻ in stoichiometric or non-stoichiometric amounts, including e.g. the dihydrochloride as well as other enantiomeres, polymorphs, mixtures and hydrates thereof. This is supported by WO 2007/014886 A1. Preferred are preparations of the monohydrochloride of (1-[(3-cyano-pyridin-2-yl)methyl]-3-methyl-7-(2-butyn-1-yl)-8-[3-(R)-amino-piperidin-1-yl]-xanthine), especially in crystalline form, preferentially in a polymorphic form as described in WO 2007/014886 A1, preferably comprising only traces of such other molecules and/or ions.

The positive effect of the monohydrochloride is documented by the experimental part of this application.

In one embodiment the pharmaceutical composition according to the invention is designed for the treatment of predominantly carnivorous non-human animals, where the predominantly carnivorous non-human animal is a predominantly carnivorous mammal, more preferred a dog (canine) or a cat (feline), even more preferred a cat (feline).

Mammals are a class of vertebrate animals whose females are characterized by the possession of mammary glands while both males and females are characterized by sweat glands, hair, three middle ear bones used in hearing, and a neocortex region in the brain. Within this class the placentals are preferred, which are characterized by the use of a placenta during gestation.

Mammals can further be divided with respect to their feeding. Some mammals feed on animal prey - this is a carnivorous diet (and includes insectivorous diets). Other mammals, called herbivores, eat plants. An omnivore eats boths prey and plants. Carnivorous mammals have a simple digestive tract, because the proteins, lipids, and minerals found in meat require little in the way of specialized digestion. Plants, on the other hand, contain complex carbohydrates, such as cellulose. The digestive tract of an herbivore is therefore host to bacteria that ferment these substances, and make them available for digestion.

The present invention is especially designed for the treatment of carnivorous or predominantly carnivorous mammals. Such mammals include especially all feliforms, such as domestic cats or big cats, and most caniforms, such as the dogs, wolves and foxes. Due to the economic importance of companion animals in modern life, the present invention is especially designed for the treatment of dogs and/or of cats, esp. of cats.

The design of pharmaceutical compositions for such patients is basically known to a person skilled in the art. This aim determines e.g. the selection of useful excipients, modes of administration, dose sizes etc. Preferred aspects according to the invention will be highlighted below.

In one embodiment the pharmaceutical composition according to the invention is designed for the treatment of a metabolic disorder and/or a metabolic disease selected from: ketoacidosis, pre-diabetes, diabetes mellitus type 1 or type 2, insulin resistance, obesity, hyperglycemia, hyperinsulinemia, elevated blood levels of fatty acids, hyperlipidemia and/or elevated blood levels of glycerol, Syndrome X, atherosclerosis, inflammation of the pancreas and/or inflammation of adipose tissue, preferably ketoacidosis, pre-diabetes and/or diabetes mellitus type 1 or type 2, more preferred diabetes mellitus type 2.

A preferred disorder and/or disease to be ameliorated by the underlying invention, is ketoacidosis. This suffering can be described as a type of metabolic acidosis which is caused by high concentrations of ketone bodies, formed by the breakdown of fatty acids and the deamination of amino acids. The two common ketones produced in humans are acetoacetic acid and β-hydroxybutyrate. In predominantly carnivorous animals, esp. cats there are mostly three ketones found: acetoacetic acid, β-hydroxybutyrate and pyruvic acid.

Ketoacidosis is an extreme and uncontrolled form of ketosis. Ketosis is a normal response to prolonged fasting. In ketoacidosis, the body fails to adequately regulate ketone production, esp. by producing Acetyl-CoA, causing such a severe accumulation of keto acids that the pH of the blood is substantially decreased. In extreme cases ketoacidosis can be fatal.

Ketoacidosis is most common in untreated type 1 diabetes mellitus, when the liver breaks down fat and proteins in response to a perceived need for respiratory substrate. Prolonged alcoholism may also lead to alcoholic ketoacidosis, which form is not of high relevance in the context of the underlying invention. Ketoacidosis can be smelled on the subject's breath. This is due to acetone, a direct byproduct of the spontaneous decomposition of acetoacetic acid.

Ketoacidosis occurs when the body is producing high levels of ketone bodies via the metabolism of fatty acids (ketosis) and the body is producing insufficient insulin to slow this production. The excess ketone bodies can significantly acidify the blood. The presence of high blood sugar levels (hyperglycemia) caused by the lack of insulin can lead to further acidity in the blood. In healthy individuals this normally does not occur because the pancreas produces insulin in response to rising ketone/blood sugar levels. Acidity results from the dissociation of the H+ ion at physiological pH of metabolic ketone bodies such as acetoacetate, acetone and β-hydroxybutyrate.

In diabetic patients, ketoacidosis is usually accompanied by insulin deficiency, hyperglycemia, and dehydration. Particularly in type 1 diabetics the lack of insulin in the bloodstream prevents glucose absorption and can cause unchecked ketone body production (through fatty acid production) potentially leading to dangerous glucose and ketone levels in the blood. Hyperglycemia results in glucose overloading the nephron and spilling into the urine (transport maximum for glucose is exceeded). Dehydration results following the osmotic movement of water into urine (Osmotic diuresis), exacerbating the acidosis.

A group of disorders and/or diseases to be ameliorated by the underlying invention, are **pre-diabetes, diabetes mellitus type 1 or type 2.** This group of sufferings has already been described above is only accentuated here.

As mentioned above the current classification divides diabetes mellitus into three classes:
(1.) Type 1 which results from the loss of function of insulin secreting cells (juvenile diabetes);
(2.) Type 2 which results from a failure of the insulin stimulated cells to respond properly to insulin stimuli, mostly after a long time of a pre-diabetes state;
(3.) secondary diabetes mellitus which can due to diabetogenic drugs or to other primary diseases.

Until now β-cells and insulin auto-antibodies have not been identified in cats, therefore type 1 diabetes is thought to be an uncommon cause of diabetes in cats (but nonetheless comprised by the aspects of the invention discussed here).

Type 2 diabetes is characterized by reduced insulin production and insulin resistance in target organs. Reduced insulin production can e.g. be caused by amyloid accumulation in β-cells, glucose toxicity, and/or pancreas infections. The defect in beta cell function is usually progressive, and in some cats and humans results in complete loss of insulin secretion. Genetic factors, glucosteroids, progesterone, lack or exercise, and obese are probable reasons for insulin resistance. For instance, in healthy cats insulin sensitivity decreases by 50% after a weight gain of >40%. Moreover, breed is recognised as a risk factor in cats (as can be seen e.g. with Burmese cats). It is thought that diabetic cats have primarily type 2, based on the fact that most diabetic cats have islet amyloid which has been called the hallmark of type 2 diabetes.

It is thought that only a substantial minority of cats have a secondary form of diabetes mellitus.

Clinical signs of diabetes mellitus, observed with cats are polydipsia, polyuria, weight loss, and/or polyphagia. In cats anorexia is more often described as polyphagia. Pathognomonic for diabetes mellitus in cats is a plantigrade stance (weakness in hind legs, hocks touch the ground when the cat walks). This is caused by a diabetic neuropathy. Vision problems and cataracts commonly seen with diabetes mellitus in dogs are seldomly found in cats.

Unfortunately, there are no internationally agreed criteria for diagnosis of diabetes mellitus in cats. However, for the purposes of the invention disclosed here, the diagnosis of diabetes mellitus is based on the identification of appropriate clinical signs, esp. of hyperglycemia and of glycosuria.

Hyperglycemia in cats is defined as plasma glucose values above normal values (2.6 - 8.4 mmol/I or 46.8 - 151.3 mg/dl). Glycosuria in cats is defined as glucose levels in urine above normal values (< 25.95 mmol/I or <290 mg/dl). The renal threshold is approximately 14 - 16 mmol/I or 250 to 288 mg/dl.

In order to document both persistent hyperglycaemia and glycosuria, it is preferred to establish a diagnosis of diabetes mellitus, because hyperglycaemia differentiates diabetes mellitus from primary renal glycosuria, whereas glycosuria differentiates diabetes mellitus from other causes of hyperglycaemia. Transient, stress-induced hyperglycemia is a common problem in cats and can cause the blood glucose concentration to increase above 300 mg/dl. Glycosuria usually does not develop in cats with stress hyperglycemia because the transient increase in the blood glucose concentration prevents glucose from accumulating in urine to a detectable concentration. However, hyperglycemia and glycosuria can occur secondary to stress in cats.

Mild hyperglycemia (i.e. up to 180 mg/dl) is clinically silent and is usually an unexpected and unsuspected finding.

Further preferred the diagnosis of feline diabetes mellitus is based on three criteria:
(1.) Two fasting blood glucose concentration measurements > 250 mg/dl;
(2.) glycosuria as defined above; and
(3.) one or more of the following: polyuria, polydipsia, polyphagia, weight loss despite good appetite, or ketonuria (without signs of severe ketoacidosis).

In addition to the above mentioned diagnostics and in order to support them, further examinations can include haematology, blood chemistry, x-ray and/or abdominal ultrasound.

The goal of diabetes therapy of pre-diabetes and other relevant forms of diabetes of predominantly carnivorous animals, esp. cats according to the invention, is the elimination of owner-observed signs (e.g. polyuria, polydypsia, anorexia, polyphagia, etc.) that occur secondary to hyperglycemia and glyosuria.

Positive results of the treatment according to the invention, especially with respect to the treatment of cats, are illustrated by the examples of this application, without limiting the scope of the invention.

PK/PD studies to measure the effect of different doses of 1-[(3-cyano-pyridin-2-yl)methyl]-3-methyl-7-(2-butyn-1-yl)-8-[3-(R)-amino-piperidin-1-yl]-xanthine CL on DPP IV activity, are presented in the experimental part of this application. In summary, doses of more than 0.1 mg/kg have been found to lead to a significant DPP IV inhibition. The goal of more than 70% DPP IV inhibition in 24 h was achieved with 1 to 3 mg/kg (-3 to 9 mg per cat, or 15 to 30 nmol/I in cat plasma). Larger degrees of DPP IV inhibition, e.g. more than 80% in 24 h can be reached by increased doses. The DPP IV inhibition duration can be prolonged with higher dosages.

Further in the context of the underlying invention, oral glucose tolerance tests (OGTT) have been successfully applied to obese and non-obese cats. Additionally to an effect of a compound relevant for the invention with regard to DPP IV inhibition, they demonstrate an effect of such a compound stimulating the activity of GLP-1, which stimulatory effect cannot be explained mechanistically at the time being.

A preferred disorder and/or disease to be ameliorated by the underlying invention, is **insulin resistance** (IR). This suffering can be described as the condition in which normal amounts of insulin are inadequate to produce a normal insulin response from fat, muscle and liver cells. Insulin resistance in fat cells reduces the effects of insulin and results in elevated hydrolysis of stored triglycerides in the absence of measures which either increase insulin sensitivity or which provide additional insulin. Increased mobilization of stored lipids in these cells elevates free fatty acids in the blood plasma. Insulin resistance in muscle cells reduces glucose uptake (and so local storage of glucose as glycogen), whereas insulin resistance in liver cells results in impaired glycogen synthesis and a failure to suppress glucose production. Elevated blood fatty acid levels (associated with insulin resistance and diabetes mellitus Type 2), reduced muscle glucose uptake, and increased liver glucose production all contribute to elevated blood glucose levels. High plasma levels of insulin and glucose due to insulin resistance are believed to be the origin of metabolic syndrome and type 2 diabetes, including its complications.

Insulin resistance is often found in individuals with visceral adiposity, hypertension, hyperglycemia and dyslipidemia involving elevated triglycerides, small dense low-density lipoprotein (sdLDL) particles, and decreased HDL cholesterol levels. With respect to visceral adiposity, a great deal of evidence suggests two strong links with insulin resistance. First, unlike subcutaneous adipose tissue, visceral adipose cells produce significant amounts of proinflammatory cytokines such as tumor necrosis factor-alpha (TNF-a), and Interleukins-1 and -6, etc. In numerous experimental models, these proinflammatory cytokines profoundly disrupt normal insulin action in fat and muscle cells, and may be a major factor in causing the whole-body insulin resistance observed in patients with visceral adiposity. A great deal of attention into the production of that enhances transcription of cytokine genes. Second, visceral adiposity is related to an accumulation of fat in the liver, a condition known as nonalcoholic fatty liver disease (NAFLD). The result of NAFLD is an excessive release of free fatty acids into the bloodstream (due to increased lipolysis), and an increase in hepatic glucose production, both of which have the effect of exacerbating peripheral insulin resistance and increasing the likelihood of Type 2 diabetes mellitus.

The cause of the vast majority of cases of insulin resistance remains unknown. There is clearly an inherited component. However, there are some grounds for suspecting that insulin resistance is related to a high-carbohydrate diet. Inflammation also seems to be implicated in causing insulin resistance.

A preferred disorder and/or disease to be ameliorated by the underlying invention, is **obesity.** This suffering can be described as a medical condition in which excess body fat has accumulated to the extent that it may have an adverse effect on health, leading to reduced life expectancy.

Obesity is associated with many diseases, particularly heart disease, type 2 diabetes, breathing difficulties during sleep, certain types of cancer, and osteoarthritis. Obesity is most commonly caused by a combination of excessive dietary calories, lack of physical activity, and genetic susceptibility, though a limited number of cases are due solely to genetics, medical reasons or psychiatric illness.

For humans, obesity is a leading preventable cause of death worldwide. And its relevance for companion animals is growing. The primary treatment for obesity is dieting and physical exercise. If this fails, anti-obesity drugs may be taken to reduce appetite or inhibit fat absorption. In severe cases, surgery is performed or an intragastric balloon is placed to reduce stomach volume and or bowel length, leading to earlier satiation and reduced ability to absorb nutrients from food. It is an aim of the invention described here to avoid such treatments for the respective animals, which in most cases would be estimated as too costly for the treatment of a companion animal. An anti-obesity drugs and/or use according to the invention is thus a cost-effective alternative for keeping the respective animals healthy.

A preferred disorder and/or disease to be ameliorated by the underlying invention, is **hyperglycemia (hyperglycaemia,** or **high blood sugar).** This suffering can be described as a condition in which an excessive amount of glucose circulates in the blood plasma. For humans, this is generally a blood glucose level of 10+ mmol/L (180 mg/dl), but symptoms may not start to become noticeable until later numbers like 15-20+ mmol/L (270-360 mg/dl)or 15.2-32.6 mmol/L. However, chronic levels exceeding 125 mg/dl can produce organ damage. For cats or other predominantly carnivorous animals, the respective critical values still remain to be defined. But there is no doubt that basically the same phenomena can be ascribed to elevated and high blood sugar levels for such patients as well.

Chronic hyperglycemia that persists even in fasting states is most commonly caused by diabetes mellitus, and in fact chronic hyperglycemia is the defining characteristic of the disease. Intermittent hyperglycemia may be present in prediabetic states. Acute episodes of hyperglycemia without an obvious cause may indicate developing diabetes or a predisposition to the disorder. In diabetes mellitus, hyperglycemia is usually caused by low insulin levels and/or by resistance to insulin at the cellular level, depending on the type and state of the disease. Low insulin levels and/or insulin resistance prevent the body from converting glucose into glycogen (a starch-like source of energy stored mostly in the liver), which in turn makes it difficult or impossible to remove excess glucose from the blood. With normal glucose levels, the total amount of glucose in the blood at any given moment is only enough to provide energy to the body for 20-30 minutes, and so glucose levels must be precisely maintained by the body's internal control mechanisms. When the mechanisms fail in a way that allows glucose to rise to abnormal levels, hyperglycemia is the result.

Other causatives for hyperglycemia esp. of humans are certain medications and critical diseases like stroke and myocardial infarction. These might also occur at animal patients.

Hyperglycemia also occurs naturally during times of infection and inflammation. When the body is stressed by these ways, endogenous catecholamines are released that - amongst other causes - serve to raise the blood glucose levels. The amount of increase varies from individual to individual and from inflammatory response to response. As such, no patient with first-time hyperglycemia should be diagnosed immediately with diabetes if that patient is concomitantly ill with something else. Further testing, such as a fasting plasma glucose, random plasma glucose, or two-hour postprandial plasma glucose level, must be performed.

Temporary hyperglycemia is often benign and asymptomatic. Blood glucose levels can rise well above normal for significant periods without producing any permanent effects or symptoms. However, chronic hyperglycemia at levels more than slightly above normal can produce a very wide variety of serious complications over a period of years, including kidney damage, neurological damage, cardiovascular damage, loss of vision, etc. In diabetes mellitus (by far the most common cause of chronic hyperglycemia), treatment according to the invention aims at maintaining blood glucose at a level as close to normal as possible, in order to avoid these serious long-term complications. Acute hyperglycemia involving glucose levels that are extremely high is a medical emergency and can rapidly produce serious complications (such as diabetic ketoacidosis). It is most often seen in persons who have uncontrolled insulin-dependent diabetes.

A preferred disorder and/or disease to be ameliorated by the underlying invention, is **hyperinsulinemia (hyperinsulinaemia)**. This suffering can be described as a condition in which there are excess levels of circulating insulin in the blood. It commonly presents in patients with diabetes mellitus type 2 or insulin resistance and is often associated with metabolic syndrome.

Most common causes of hyperinsulinemia are Diabetes mellitus type 2 and Insulin resistance.

The most severe effects of hyperinsulinemia are increased synthesis of VLDL (hypertriglyceridemia), Hypertension (insulin increases sodium retention by the renal tubules) and Coronary Artery Disease (increased insulin damages endothelial cells). It might also lead to hypoglycemia.

Further preferred disorders and/or diseases to be ameliorated by the underlying invention, are **elevated blood levels of fatty acids, hyperlipidemia and/or elevated blood levels of glycerol**. These disorders and/or diseases might be detected as sufferings by themselves or as clinical signs of other metaboloic diseases, esp. those described above.

Blood lipids (fatty molecules or blood fats) are lipids in the blood, either free or bound to other molecules. They are mostly transported in a protein capsule, and the density of the lipids and type of protein determines the fate of the particle and its influence on metabolism. The concentration of blood lipids depends on intake and excretion from the intestine, and uptake and secretion from cells. Blood lipids are mainly fatty acids and cholesterol.

Hyperlipidemia (hyperlipoproteinemia, dyslipidemia or hyperlipidaemia) is the presence of raised or abnormal levels of lipids and/or lipoproteins in the blood.

Lipid and lipoprotein abnormalities are extremely common in the general population, and are regarded as a highly modifiable risk factor for cardiovascular disease due to the influence of cholesterol, one of the most clinically relevant lipid substances, on atherosclerosis. In addition, some forms may predispose to acute pancreatitis.

Glycerol is a precursor for synthesis of triacylglycerols and of phospholipids in the liver and adipose tissue. When the body uses stored fat as a source of energy, glycerol and fatty acids are released into the bloodstream after hydrolysis of the triglycerides. The glycerol component can be converted to glucose by the liver and provides energy for cellular metabolism.

Before glycerol can enter the pathway of glycolysis or gluconeogenesis (depending on physiological conditions), it must be converted to their intermediate glyceraldehyde 3-phosphate.

Raised levels of glycerol in the blood my lead to obesity and/or an increased risk of developing type 2 diabetes mellitus.

Normal levels of free fatty acids in the blood of companion animals are 50 to 100 mg/dl of triglycerides (0.6 to 1.2 mmol/l). Normal levels of cholesterol in the blood of companion animals are 120-400 mg/dl for the dog, and 70-150 mg/dl for the cat. Accordingly, hyperlipidemia and elevated levels of glycerol for these patients, are characterized by concentration values above. It is an aim of the underlying invention to keep these values at a moderate, at least sub-clinical level.

A preferred disorder and/or disease to be ameliorated by the underlying invention, is **Syndrome X.** This suffering can be described as a combination of medical disorders that increase the risk of developing cardiovascular disease and diabetes. Metabolic syndrome is also known as metabolic syndrome X, syndrome X, insulin resistance syndrome, Reaven's syndrome, and CHAOS (as an abbreviation for Coronary artery disease, Hypertension, Atherosclerosis, Obesity, and Stroke).

The exact mechanisms of the complex pathways of metabolic syndrome are not yet completely known. The pathophysiology is extremely complex and has been only partially elucidated. Most patients are older, obese, sedentary, and have a degree of insulin resistance. The most important factors in order are: (1.) overweight and obesity, (2.) genetics, (3.) aging, and (4.) sedentary lifestyle, i.e., low physical activity and excess caloric intake.

A further risk factor is diabetes mellitus. It is estimated that the large majority (~75%) of human patients with type 2 diabetes or impaired glucose tolerance (IGT) have the metabolic syndrome.

The pathophysiology is commoly characterized by the development of visceral fat after which the adipocytes (fat cells) of the visceral fat increase plasma levels of TNFα and alter levels of a number of other substances (e.g., adiponectin, resistin, PAI-1). TNFα has been shown not only to cause the production of inflammatory cytokines, but possibly to trigger cell signalling by interaction with a TNFα receptor that may lead to insulin resistance.

Current first line treatment is change of lifestyle (i.e., caloric restriction and physical activity). However, drug treatment is frequently required. Generally, the individual disorders that comprise the metabolic syndrome are treated separately. Diuretics and ACE inhibitors may be used to treat hypertension. Cholesterol drugs may be used to lower LDL cholesterol and triglyceride levels, if they are elevated, and to raise HDL levels if they are low. It is preferred to support and/or base these treatments on a pharmaceutical composition according to the invention discussed here.

A preferred disorder and/or disease to be ameliorated by the underlying invention, is **atherosclerosis.** This suffering can be described as a condition in which an artery wall thickens as the result of a build-up of fatty materials such as cholesterol. It is a syndrome affecting arterial blood vessels, a chronic inflammatory response in the walls of arteries, in large part due to the accumulation of macrophage white blood cells and promoted by low density (especially small particle) lipoproteins (plasma proteins that carry cholesterol and triglycerides) without adequate removal of fats and cholesterol from the macrophages by functional high density lipoproteins (HDL), (see apoA-1 Milano). It is commonly referred to as a hardening or furring of the arteries. It is caused by the formation of multiple plaques within the arteries.

Atherosclerosis, though typically asymptomatic for a long time, eventually produces two main problems: First, the atheromatous plaques, though long compensated for by artery enlargement, eventually lead to plaque ruptures and clots inside the artery lumen over the ruptures. The clots heal and usually shrink but leave behind stenosis (narrowing) of the artery (both locally and in smaller downstream branches), or worse, complete closure, and, therefore, an insufficient blood supply to the tissues and organ it feeds. Second, if the compensating artery enlargement process is excessive, then a net aneurysm results.

These complications of advanced atherosclerosis are chronic, slowly progressive and cumulative. Most commonly, soft plaque suddenly ruptures (see vulnerable plaque), causing the formation of a thrombus that will rapidly slow or stop blood flow, leading to death of the tissues fed by the artery in approximately 5 minutes. This catastrophic event is called an infarction. One of the most common recognized scenarios is called coronary thrombosis of a coronary artery, causing myocardial infarction (a heart attack). Even worse is the same process in an artery to the brain, commonly called stroke. Another common scenario in the very advanced disease is claudication from insufficient blood supply to the legs, typically due to a combination of both stenosis and aneurysmal segments narrowed with clots. Since atherosclerosis is a body-wide process, similar events occur also in the arteries to the brain, intestines, kidneys, legs, etc.

Metabolic diseases, esp. diabetes, dyslipidaemia and elevated serum levels of triglycerides and/or of insulin are risk factors for the development of atherosclerosis, besides behavioural risk factors like food preferences. Accordingly, a composition according to the invention is helpful to minimize an individual's propensity to develop atherosclerosis, esp. in the context of other risk factors which the individual is not able to change.

A preferred disorder and/or disease to be ameliorated by the underlying invention, is **an inflammation of the pancreas (pancreatitis)**. In humans this suffering is described to occur in two very different forms. Acute pancreatitis is sudden while chronic pancreatitis is characterized by recurring or persistent abdominal pain with or without steatorrhea or diabetes mellitus.

Excessive alcohol use is often cited as the most common cause of acute pancreatitis, but of low relevance in veterinary medicine. Other known causes include hypertriglyceridemia (but not hypercholesterolemia) and only when triglyceride values exceed 1500 mg/dl (16 mmol/l), hypercalcemia, viral infection, trauma, vasculitis (i.e. inflammation of the small blood vessels within the pancreas), and autoimmune pancreatitis.

Metabolic diseases, esp. dyslipidaemia and elevated serum levels of triglycerides are risk factors for the development of pancreatitis. Accordingly, a composition according to the invention is helpful to minimize an individual's propensity to develop pancreatitis.

A preferred disorder and/or disease to be ameliorated by the underlying invention, is **an inflammation of adipose tissue (panniculitis)**. This suffering can be described as a group of diseases whose hallmark is inflammation of subcutaneous adipose tissue (the fatty layer under the skin).

It can occur in any fatty tissue (cutaneous or visceral) and is often diagnosed on the basis of a deep skin biopsy, and can be further classified by histological characteristics based on the location of the inflammatory cells (within fatty lobules or in the septa which separate them) and on the presence or absence of vasculitis. Panniculitis can also be classified based on the presence or absence of systemic symptoms.

Metabolic diseases and esp. pancreatitis are risk factors for the development of panniculitis. Accordingly, a composition according to the invention is helpful to minimize an individual's propensity to develop panniculitis.

It is preferred that a pharmaceutical composition according to the invention exerts more than one of these medical effects.

In one embodiment the pharmaceutical composition according to the invention comprises pharmaceutically acceptable excipients, preferably a carrier or vehicle for the pharmaceutically active compound.

These pharmaceutically active compounds have already been explained above.

In one embodiment the pharmaceutical composition according to the invention is a solid formulation. Such a formulation can be a tablet, for example.

Solid formulations according to the invention comprise preferably carriers and/or disintegrants selected from the group of sugars and sugar alcohols, e.g. mannitol, lactose, starch, cellulose, microcrystalline cellulose and cellulose derivatives, e. g. methylcellulose, and the like.

One or several binders according to the invention are preferably selected from the group consisting of polyvidone (used synonymously for povidone), methylcellulose, hydroxypropylmethylcellulose (HPMC), hydroxymethylcellulose, starch, gelatine, and the like.

The solid formulation according to the invention may also comprise one or several flow regulators selected from the group consisting of silica, preferably colloidal anhydrous silica, calcium silicate, magnesium silicate, talc, and the like.

The solid formulation according to the invention may also comprise one or several disintegrants selected from the group consisting of croscarmellose sodium, sodium starch glycolat, pregelatinised starch, cross-linked polyvinylpyrrolidone and the like.

The solid formulation according to the invention may also comprise one or several lubricants selected from the group consisting of magnesium stearate, calcium stearate, glyceryl behenate, polyethylene glycol, stearic acid, talc and the like.

The invention preferably relates to a solid formulation according to the invention, characterized in that the carriers are mannitol, starch and/or lactose. The carrier material can consist of coarse particles greater than 200 µm in size, equal or smaller than 200 µm in size, or spray-dried material. Preferably the carrier material consists of coarse particles greater than 200 µm in size.

The invention preferably also relates to a solid formulation according to the invention, characterized in that the starch or various starches are selected from the group consisting of corn starch, native starch, gelatinized starch, partly gelatinized starch, starch powder, starch granules, chemically modified starch and swellable physically modified starch.

A preferred solid pharmaceutical composition according to the invention is characterized in that the weight of the whole solid formulation of one dose is in the range of 50 to 3000 mg, preferred 75 to 1000 mg, more preferred 80 to 500 mg, even more preferred 85 to 125 mg, and most preferred 90 to 250 mg.

Such a solid composition might be prepared in way that is basically known to a person skilled in the art.

Such manufacturing procedures comprise direct compression, dry granulation and wet granulation. In the direct compression process, the active ingredient according to the invention and all other excipients are placed together in a compression apparatus that is directly applied to press tablets out of this material. The resulting tablets can optionally be coated afterwards in order to protect them physically and/or chemically, e.g. by a material known from the state of the art.

Dry granulation processes create granules by light compaction of the powder blend under low pressures. The compacts so-formed are broken up gently to produce granules (agglomerates). Dry granulation can be conducted on a tablet press using slugging tooling or on a roll press called a roller compactor. Dry granulation equipment offers a wide range of pressures to attain proper densification and granule formation. Dry granulation is simpler than wet granulation (see below), therefore the cost is reduced. However, dry granulation often produces a higher percentage of fine granules, which can compromise the quality or create yield problems for the tablet. Advantageously a 'dry binder' can be added to the formulation to facilitate the formation of granules. Also these resulting granules can optionally be coated afterwards in order to protect them.

Preferred is a wet granulation process, esp. a fluid-bed granulation process. More preferred is a fluid-bed granulation process comprising or consisting of the steps:
a) an aqueous solution of 1-[(3-cyano-pyridin-2-yl)methyl]-3-methyl-7-(2-butyn-1-yl)-8-[3-(R)-amino-piperidin-1-yl]-xanthine (in any suitable chemical form) and a binder as defined above is sprayed onto a solid carrier bed comprising one or several carriers and/or excipients as defined above, flavor and
b) the mixture of a) is dried and
c) the mixture of b) is sieved and de-agglomerated and
d) a flow regulator is added to the mixture of c) and
e) a lubricant is added to the mixture of d) and
f) the mixture of e) is blended for uniformity of granules to obtain fina granules and/or
g) the final granules of f) are compressed to solid formulations.

Step g) can be omitted, esp. when the solid formulation is a granule. If the solid formulation is a tablet, step g) is preferably carried out. A further coating step as described above can also be applied.

Therefore, the solid formulation according to the invention preferably is a granule (or a plurality of such granules) or a tablet. The administration of the solid formulation can take place by mixing with food or by offering the granules directly to the animal, e. g. in a bowl. The application of the granular form will allow an individual dosing according to the body weight of the animal.

The invention preferably also relates to a tablet according to the invention, characterized in that the tablet is stable for at least 18 months at 25°C and 60% relative humidity. This might be reached by a combination of the above mentioned excipients basically accessible for a person skilled in the art.

Suitable packaging materials for tablets according to the invention are selected from, but not limited to: aluminum/aluminum blisters, PVC/PVDC blisters, and HDPE (high density polyethylene bottles).

The invention preferably also relates to a tablet according to the invention, characterized in that the tablet is oblong in shape. For such a tablet, characteristics like crushing strength, disintegration, uniformity of weight and content uniformity fulfill the requirements of the European Pharmacopoeia (ISBN/ISSN 92-871-5106-7 of 4t Edition 2004, Vol. 4.8, European Directorate for the Quality of Medicines (EDQM), European Pharmacopoeia, 226 avenue de Colmar, F-67029 Strasbourg, France, http ://www. pheur. org) and the United States Pharmacopoeia (http: //www. usp. oro : in print: USP-NF, catalog No. 2270001).

A typical formulation of a composition according to the invention is a solid formulation, and most preferred a tablet, characterized in that the solid formulation or tablet comprises 0.5 to 20 mg, increasingly preferred 1 to 40 mg, 1.25 mg to 30 mg, 2.5 mg to 20 mg and 5 mg to 10 mg of active substance of a compound relevant for the invention, including any other value in between.

Further excipients are, preferably mannitol (35-50 % by weight relative to the dry mass of the solid formulation/tablet = (w/w)), corn starch (25-50 % w/w), croscarmellose-sodium (1-5 %), artificial beef flavor (5-30 % w/w), polyvidone (1-5 % w/w), colloidal anhydrous silica (0.1-1, preferably 0.1-0. 5 % w/w) and magnesium stearate (0, 25-1, 5 % w/w), wherein the percentage by weight of active substance contains preferably about 0, 25% (w/w) and the sum of the percentages by weight of all ingredients of the solid formulation including active substance is 100 % (w/w).

It is preferred that such pharmaceutically acceptable excipients ameliorate the chewability and/or the palatability of the composition, especially the chewability and/or the palatability for a dog and/or a cat, more preferred the chewability and/or the palatability for a cat.

The composition according to the invention might be applied in all forms acceptable with respect to the specific purpose. However, it has been proven useful that veterinary formulations of pharmaceuticals are added to the meals of the respective animals or are administered separately by an oral preparation that might be accepted by the animal. For carnivorous animals such formulations of additives or of separate preparations resemble their usual feed, e.g. with respect to its haptic appearance or its taste or smell. For this purpose they might be tough enough to animate a dog or a cat to gnaw.

Flavors are preferably selected from artificial beef flavours, artificical chicken flavours, pork liver extract, artificial meat flavour, honey flavour and the like.

In one embodiment the pharmaceutical composition according to the invention is designed for oral or parenteral administration, preferably for oral administration.

Especially the oral administration is ameliorated by excipients which modify the smell and/or haptic appearance of the pharmaceutical composition for the intended patient as described before.

It is also enviseaged that the composition according to the invention be designed to be added to the normal food of the respective animal. Therefore it preferably has a respective colour, smell, taste and/or haptic appearance in order to resemble the food of the animal and thus to be not or nearly not perceptible by the patient.

Also liquid preparations are comprised by the invention which can be added dropwise to the drinking water of the animal or to the respective food. It is an advantage of such a dosage form that it allows a very precise dosing. Especially this dosage form is characterized by a respective taste.

In one embodiment the pharmaceutical composition according to the invention is designed for the administration of 1 to 5 doses per day, preferably 1 or 2 doses per day, more preferred 1 dose per day.

The size of the doses as well as the mode of application depend on the concentration of the active ingredient on the one hand and on its bio-availability and degradation time in the patient on the other hand. Especially carnivorous animals are characterized by a rapid gut passage which makes it advantageous to develop retard compositions.

An administration of the composition according to the invention at moderate intervals over the day is also preferred with respect to the acceptance of the treatment. In this respect it is preferred that the number of necessary treatments can be adapted to the typical feeding rates of the animal, even more preferred at only one dose per day.

The development of such pharmaceutical compositions is basically known for a person skilled in the art and can be reached by a combination of the excipients listed above. Protective layers esp. those which dissolve in certain parts of the gut are further means in order to reach this aim.

In one embodiment the pharmaceutical composition according to the invention is designed for the administration of 0.1 to 100 mg of the active ingredient per kg per day, preferably of 0.5 to 50 mg/kg per day, more preferred of 0.75 to 25 mg/kg per day, even more preferred of 1.0 to 15 mg/kg per day.

In one embodiment the pharmaceutical composition according to the invention is designed for reaching a blood plasma concentration of the active ingredient within the range of 6 to 10 nmol per I.

It is prefered to keep such levels over a time interval of 24 h, especially by retard formulations to be applied preferably once per day, as described above.

In one embodiment the pharmaceutical composition according to the invention comprises another pharmaceutically effective compound which is effective against a metabolic disorder or metabolic disease, preferably against ketoacidosis, pre-diabetes, diabetes mellitus type 1 or type 2, insulin resistance, obesity, hyperglycemia, hyperinsulinemia, elevated blood levels of fatty acids, hyperlipidemia and/or elevated blood levels of glycerol, Syndrome X, atherosclerosis, inflammation of the pancreas and/or inflammation of adipose tissue, more prefered against ketoacidosis, pre-diabetes and/or diabetes mellitus type 1 or type 2, even more preferred against diabetes mellitus type 2.

Such compounds are described in the art and can be incorporated into respective pharmaceutical compositions by a person skilled in the are by methods which are basically known.

A focus lies on the combination of a compound according to the invention with one or more oral hypoglycaemic compounds: Several such oral hypoglycaemic drugs are described and/or already approved for the treatment of type 2 diabetes mellitus in humans. Mechanisms exerted by such drugs are for example the stimulation of pancreatic insulin secretion in a glucose-independent manner (sulfonylureas, meglitinides), the stimulation of pancreatic insulin secretion in a glucose-dependent manner (other DPP IV inhibitors), enhancement of the tissue sensitivity to insulin (biguanides, thiazolidinediones), or slowing the postprandial intestinal glucose absorption (alpha-glucosidase inhibitors).

Oral medications like Glipizide (sulfonylurea) are described to work in some small proportion of cats (see above), however, accompanied with adverse site effects such as vomiting and icterus and even further damages. These effects reduce the chances of remission. They have also been shown to cause liver damage if they are applied alone.

Therefore it is advantageous to combine such compounds with one compound according to the invention, esp. when both compounds work via different biochemical mechanisms. In such a combined treatment, at least one compound, preferably both compounds (1-[(3-cyano-pyridin-2-yl)methyl]-3-methyl-7-(2-butyn-1-yl)-8-[3-(R)-amino-piperidin-1-yl]-xanthine and the other compound) can be applied at lower concentrations or at a less rigid application scheme than applied alone but showing positive results by their combined or even synergistic effect. Negative side effects like those described above can be lowered or even elimininated by such a combined application.

In one embodiment such a pharmaceutical composition according to the invention comprises one or more compounds selected from SGLT-2 inhibitors.

SGLT-2 is the abbreviation for "sodium dependent glucose transporter 2", a protein naturally expressed in the kidney and responsible for trans-membrane glucose transport. It is believed that chemical compounds interfering with the natural activity of this factor lead to an enhanced excretion of blood glucose via the kidney into the individual's urine. This principle is applied to the therapy of metabolic diseases, esp. of diverse forms of diabetes.

This mechanism is quite different to the inhibition of DPP-4, exerted by 1-[(3-cyano-pyridin-2-yl)methyl]-3-methyl-7-(2-butyn-1-yl)-8-[3-(R)-amino-piperidin-1-yl]-xanthine. Accordingly it is an aim of this aspect of the invention to combine both positive effects for an effective treatment of the metabolic diseases listed above, especially of pre-diabetes, diabetes mellitus type 1 or type 2 and insulin resistance, more preferred diabetes mellitus type 2.

Such a combination is especially preferred as far as it leads to a synergistic effect of both activity profiles. They can be administered simultaneously or timely staggered. A simultaneous administration is preferred, esp. via a combination product comprising pharmaceutically active ingredients belonging to both groups.

Useful SGLT-2 inhibitors and pharmaceutical compositions comprising them are disclosed for example by WO 2005/092877 A1 and WO 2007/093610 A1. WO 2005/092877 A1 discloses certain glucopyranosyl-substituted benzol derivatives as well as drugs containing said compounds, the use thereof and methods for the production thereof. WO 2007/093610 A1 discloses certain glucopyranosyl-substituted benzonitrile derivatives along with drugs containing said compounds, the use thereof and methods for the production thereof. All subject matter disclosed therein is incorporated herewith. The selection of certain compounds is mostly preferred if a synergy can be reached by a combination with 1-[(3-cyano-pyridin-2-yl)methyl]-3-methyl-7-(2-butyn-1-yl)-8-[3-(R)-amino-piperidin-1-yl]-xanthine, esp. with respect to one of the above mentioned aspects of the invention.

Especially useful SGLT-2 inhibitors are selected from the group consisting of:
(1) Dapagliflozin;
(2) Remogliflozin or Remogliflozin etabonate;
(3) Sergliflozin or Sergliflozin etabonate;
(4) 1-Chloro-4-(β-D-glucopyranos-1-yl)-2-(4-ethyl-benzyl)-benzene;
(5) (1S)-1,5-Anhydro-1-[5-(azulen-2-ylmethyl)-2-hydroxyphenyl]-D-glucitol;
(6) (1S)-1,5-Anhydro-1-[3-(1-benzothien-2-ylmethyl)-4-fluorophenyl]-D-glucitol;
(7) Thiophen derivative of the formula (7-1) 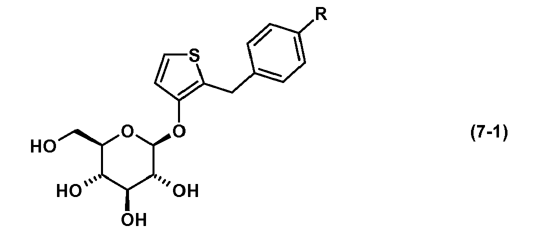 wherein R denotes methoxy or trifluoromethoxy;
(8) 1-(β-D-glucopyranosyl)-4-methyl-3-[5-(4-fluorophenyl)-2-thienylmethyl]benzene;
(9) Spiroketal derivative of the formula (9-1): 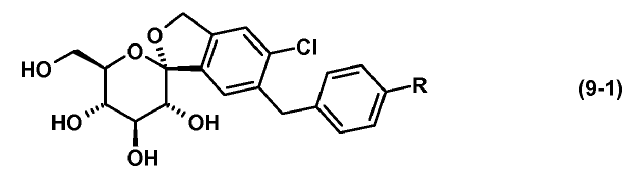 wherein R denotes methoxy, trifluoromethoxy, ethoxy, ethyl, isopropyl or tert. butyl;
(10) a glucopyranosyl-substituted benzene derivative of the formula (10-1) 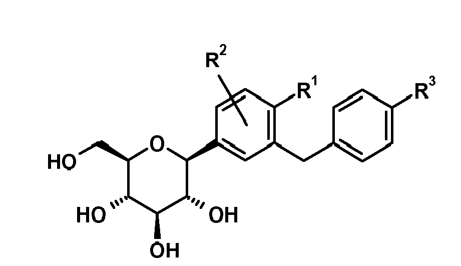 wherein R¹ denotes Cl, methyl or cyano; R² denotes H, methyl, methoxy or hydroxy and R³ denotes ethyl, cyclopropyl, ethinyl, ethoxy, (*R*)-tetrahydrofuran-3-yloxy or (*S*)-tetrahydrofuran-3-yloxy,
(11) a pyrazole-O-glucoside derivative of the formula (11-1) 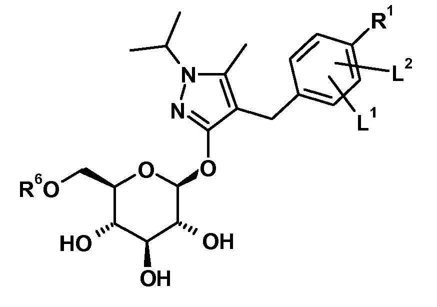 wherein
   R¹ denotes C₁₋₃-alkoxy, L¹, L² independently of each other denote H or F,
   R⁶ denotes H, (C₁₋₃-alkyl)carbonyl, (C₁₋₆-alkyl)oxycarbonyl, phenyloxycarbonyl, benzyloxycarbonyl or benzylcarbonyl.

Such especially useful SGLT-2 inhibitors comprise every pharmaceutically acceptable form thereof, especially a pharmaceutically acceptable salt, a hydrate or a solvate thereof.

Compounds belonging to the groups (1) to (9) are disclosed in WO 2009/022010 A1 and references disclosed therein, along with appropriate ways for their production as well as modes for their incorporation in pharmaceutical compositions and respective ways to treat metabolic diseases, esp. forms of diabetes.

This application further discloses that these compounds can effectively be combined with DPP IV inhibitors in order to allow a treatment or prevention of metabolic disorders and related conditions, especially one or more conditions selected from type 1 diabetes mellitus, type 2 diabetes mellitus, impaired glucose tolerance and hyperglycemia. In the context of the current invention pharmaceutical compositions comprising combinations of those compounds and a compound relevant for the invention are respectively preferred, especially with respect to the aspects explained above.

Compounds belonging to the group (10) are disclosed in WO 2009/022007 A1, along with appropriate ways for their production as well as modes for their incorporation in pharmaceutical compositions and respective ways to treat metabolic diseases, esp. forms of diabetes.

This application further discloses that these compounds can effectively be combined with DPP IV inhibitors in order to allow a treatment or prevention of metabolic disorders and related conditions, especially one or more conditions selected from type 1 diabetes mellitus, type 2 diabetes mellitus, impaired glucose tolerance and hyperglycemia. In the context of the current invention pharmaceutical compositions comprising combinations of those compounds and a compound relevant for the invention are respectively preferred, especially with respect to the aspects explained above.

Compounds belonging to the group (11) are disclosed in WO 2009/022008 A1, along with appropriate ways for their production as well as modes for their incorporation in pharmaceutical compositions and respective ways to treat metabolic diseases, esp. forms of diabetes.

This application further discloses that these compounds can effectively be combined with DPP IV inhibitors in order to allow a treatment or prevention of metabolic disorders and related conditions, especially one or more conditions selected from type 1 diabetes mellitus, type 2 diabetes mellitus, impaired glucose tolerance and hyperglycemia. In the context of the current invention pharmaceutical compositions comprising combinations of those compounds and a compound relevant for the invention are respectively preferred, especially with respect to the aspects explained above.

In one embodiment a pharmaceutical composition according to the invention is designed for a coadministration with insulin, preferably designed for a simultaneous, a sequential and/or a chronologically staggered coadministration with insulin.

The classical treatment of pre-diabetes, diabetes mellitus type 1 or type 2 is the administration of insulin. Different modes of insulin administration are established in the state of the art and can be selected with respect to the patient's individual compliance.

Especially for severe forms of diabetes and/or a combination with one or more of the other metabolic diseases with in the ambit of this treatment it is a part of the actual invention to combine any form insulin treatment with an administration of a 1-[(3-cyano-pyridin-2-yl)methyl]-3-methyl-7-(2-butyn-1-yl)-8-[3-(R)-amino-piperidin-1-yl]-xanthine comprising pharmaceutical composition as described above.

Accordingly it is an aim of this aspect of the invention to combine both positive effects for an effective treatment of the metabolic diseases listed above, especially of pre-diabetes, diabetes mellitus type 1 or type 2 and insulin resistance, more preferred diabetes mellitus type 2. Such a combination is especially preferred as far as it leads to a synergistic effect of both activity profiles.

Insulin is available in different forms. The person skilled in the art is able to choose the form that can be applied for each specific case. One preferred form of insulin to be integrated in a combination and/or combination therapy according to the invention is Protamine Zinc Recombinant Human Insulin (PZIR), e.g. described by Nelson et al. (2009), J. Vet. Intern. Med., 23, 787-793). Such an isulin is commercially available under the trade name ProZinc from Boehringer Ingelheim Vetmedica Inc., USA.

A compound relevant for the invention and insulin can be co-administered simultaneously, sequentially and/or chronologically staggered.

The best mode might be tested and selected individually. Accordingly, it lies within the ambit of the underlying invention that a veterinarian is able to chose between preparations of 1-[(3-cyano-pyridin-2-yl)methyl]-3-methyl-7-(2-butyn-1-yl)-8-[3-(R)-amino-piperidin-1-yl]-xanthine or of any form of it and/or of insulin at different forms, sizes and concentration values in order to develop the ideal individual mode of treatment for each patient.

Advantageous combinations are:
(1) 1-[(3-cyano-pyridin-2-yl)methyl]-3-methyl-7-(2-butyn-1-yl)-8-[3-(R)-amino-piperidin-1-yl]-xanthine once a day plus insulin twice a day, with the advantage that the compound relevant for the invention reduces the amount of insulin injected, resulting in better glycemic control than insulin alone; and
(2) 1-[(3-cyano-pyridin-2-yl)methyl]-3-methyl-7-(2-butyn-1-yl)-8-[3-(R)-amino-piperidin-1-yl]-xanthine once a day plus insulin once a day, with the advantage that the compound relevant for the invention reduces the frequency of insulin injections, also resulting in better glycemic control than insulin alone.

In one embodiment of the invention the pharmaceutical composition according to the invention is used for the treatment of a metabolic disorder or metabolic disease of a predominantly carnivorous non-human animal.

The advantages of such a use are equivalent to those of the pharmaceutical composition and preferred embodiments thereof, which have been explained in the foregoing description.

Preferred forms and/or advantages of such a use are:
- Reduction of hyperglycaemia and of hyperglycaemia associated clinical signs in diabetic cats;
- Reduction of hyperglycaemia in sub-clinical diabetic rats;
- Improvement in glucose tolerance in glucose impaired cats;
- Preventing the onset of diabetes mellitus;
- Demonstration of effect on disease progression (e.g. regeneration of β-cell function, i.e. increase in b-cell mass);
- Management of obesity in cats;
- Management of obesity in cats as adjunct therapy to other obesity management therapies (e.g. diet, exercise).

All of these modes target a different population of diabetic cats than insulin does currently.

*Vice versa,* a composition as defined above is designed to reach one or more of these aims, e.g. with respect to dosage forms or combination with adavatageous excipients.

Advantages of such uses are:
- Reduction of hyperglycaemia and hyperglycaemia associated clinical signs in early diabetic cats;
- Efficacy with regard to an effect on disease progression, e.g. preservation of β-cell function;
- Very safe and/or tolerable profile, especially absence/lower incidence of risk of hypoglycaemia, weight gain, or other risks identified with other non-approved human oral antidiabetics;
- Better convenience of 1-[(3-cyano-pyridin-2-yl)methyl]-3-methyl-7-(2-butyn-1-yl)-8-[3-(R)-amino-piperidin-1-yl]-xanthine comprising pharmaceutical compositions as insulin injections.

It is further preferred to combine the use according to the invention with a test for animal-specific biomarkers for detecting forms of diabetes

A use according to the invention - as far as it pertains to the treatment of diabetes - is preferably accompanied by a monitoring of the diabetic control. The basic objective of the treatment of diabetes, e.g. of a cat, according to the invention is to eliminate the clinical signs of diabetes mellitus while avoiding the common complications associated with the disease. Such complications described for cats include weakness, ataxia, plantigrade stance caused by peripheral neuropathy, weight loss, poor hair coat from lack of grooming, hypoglycaemia, recurring ketosis, poor control of glycaemia secondary to concurrent infection, inflammation, neoplasia, or hormonal disorders.

The establishment of near-normal blood glucose concentrations is preferable but - other than for human therapy - not believed to be always necessary for diabetic animals and therefore not always the goal of a therapy according to the invention. Almost all owners are happy and most cats are healthy and relatively asymptomatic if blood glucose concentrations are kept between 100 - 300 mg/dl. Such a lowering of the clinical sings of any form of diabetes or pre-diabetes is also a cost-effective mode which has the additional advantage of keeping possible side effects of the compounds according to the invention or of the combination drugs at a moderate level.

Several techniques for monitoring lie within the ambit of the actual invention: history and physical examination, fructosamine concentrations (normal range: 190 - 365 µmol/l; excellent control: 350 - 400 µmol/l; good control: 400 - 450 µmol/l; fair control: 450 to 500 µmol/l; poor control: >500 µmol/l; prolonged hypoglycaemia: < 300 µmol/l; glycated haemoglobin concentration (normal range: 0.9 - 2.5 %; excellent control: 1.0 - 2.0 %; good control: 2.0 - 2.5 %; fair control: 2.5 - 3.0 %; poor control: >3.0 %; prolonged hypoglycaemia: <1.0 %), serial blood glucose curve, protocol for generating the serial blood glucose curve at home and other techniques known to the person skilled in the art.

Diabetic remission is highly preferred and can be gained in newly diagnosed cats if treated appropriately. Remission is more likely if glycemic control is optimum, so that β-cells can recover from glucose toxicity.

This use of the pharmaceutical composition according to the invention is preferably supported by insulin administration, diet (high protein, low carbohydrate), weight loss management, stop diabetogenic treatments, exercise, other hypoglycaemic medications, and/or the avoidance or control of concurrent inflammatory, infectious, neoplastic, and hormonal disorders, all basically known to a person skilled in the art, esp. to a veterinarian.

In one embodiment of the invention 1-[(3-cyano-pyridin-2-yl)methyl]-3-methyl-7-(2-butyn-1-yl)-8-[3-(R)-amino-piperidin-1-yl]-xanthine or a pharmaceutically acceptable salt or a solvate thereof is used for the preparation of a pharmaceutical composition for the treatment of a metabolic disorder or metabolic disease of a predominantly carnivorous non-human animal.

All specific embodiments, details and advantages of respectively preferred forms within the ambit of this aspect of the invention, can be deduced from the above explanations accordingly and apply to this aspect *mutatis mutandis.*

Preferably such a use comprises the respective combined use of one or more additional compounds selected from SGLT-2 inhibitors, preferably selected from the group defined above.

The preferred combinations and advantages thereof as described above, apply to this aspect of the invention *mutatis mutandis.*

In one embodiment of the invention 1-[(3-cyano-pyridin-2-yl)methyl]-3-methyl-7-(2-butyn-1-yl)-8-[3-(R)-amino-piperidin-1-yl]-xanthine or a pharmaceutically acceptable salt or a solvate thereof is used for the treatment of a metabolic disorder or metabolic disease of a predominantly carnivorous non-human animal.

All specific embodiments, details and advantages of respectively preferred forms within the ambit of this aspect of the invention, can be deduced from the above explanations accordingly and apply to this aspect *mutatis mutandis.*

Preferably such a use comprises the respective combined use of one or more additional compounds selected from SGLT-2 inhibitors, preferably selected from the group defined above.

The preferred combinations and advantages thereof as described above, apply to this aspect of the invention *mutatis mutandis.*

The following examples are intended to illustrate the underlying invention in more detail without any limitation of the scope of the claims.

### EXAMPLES

### Example 1

### In vitro selectivity of 1-[(3-cyano-pyridin-2-yl)methyl]-3-methyl-7-(2-butyn-1-yl)-8-[3-(R)-amino-piperidin-1-yl]-xanthine

In order to determine the in vitro selectivity of 1-[(3-cyano-pyridin-2-yl)methyl]-3-methyl-7-(2-butyn-1-yl)-8-[3-(R)-amino-piperidin-1-yl]-xanthine, the ability of this compound to inhibit a number of human proteases was investigated as described in Thomas et al. (2008), J. Pharmacol. Exp. Ther., vol. 325, p. 175-182, chapter In vitro DPP-4 Inhibition Assay, p. 176.

1-[(3-cyano-pyridin-2-yl)methyl]-3-methyl-7-(2-butyn-1-yl)-8-[3-(R)-amino-piperidin-1-yl]-xanthine monohydrochloride, up to 100 µM, did not exert any inhibitory effect on plasmin, thrombin, trypsin, DPP-II, Prolyl-Oligo Peptidase (or Prolyl Endopeptidase), alanine-amino-peptidase or amino-peptidase P. DPP 8 and DPP 9 were inhibited with IC50 values of 95 and greater than 10 µM respectively.

In selectivity assays as described in Dörje et al. (1991), J. Pharmacol. Exp. Ther., vol. 256, p. 727-733, chapter Radio ligand binding studies, p. 729, using the full panel of receptors, ion channels and enzymes, the only interaction observed with 1-[(3-cyano-pyridin-2-yl)methyl]-3-methyl-7-(2-butyn-1-yl)-8-[3-(R)-amino-piperidin-1-yl]-xanthine monohydrochloride was with M1 muscarinic receptors, with IC50 in a binding assay of 3 µM. These in vitro profiling data demonstrate that 1-[(3-cyano-pyridin-2-yl)methyl]-3-methyl-7-(2-butyn-1-yl)-8-[3-(R)-amino-piperidin-1-yl]-xanthine is a potent, selective, reversible and competitive inhibitor of DPP IV enzyme.

### Example 2

**The effect of 1-[(3-cyano-pyridin-2-yl)methyl]-3-methyl-7-(2-butyn-1-yl)-8-[3-(R)-amino-piperidin-1-yl]-xanthine on plasma DPP IV activity after oral administration**

The effect of 1-[(3-cyano-pyridin-2-yl)methyl]-3-methyl-7-(2-butyn-1-yl)-8-[3-(R)-amino-piperidin-1-yl]-xanthine on plasma DPP IV activity after oral administration was investigated in the rat, the Beagle dog and the Rhesus monkey. These investigations were conducted as described in the following.

Male HanWistar rats (Crl:WI(Han) were obtained from Charles River (Germany). Animals were fed ad libitum with a standard pelleted diet (Diet No. 3438, Provimi Kliba, Switzerland) and had free access to water. Animals were housed in groups with a 12 h/12 h light/dark cycle (lights out between 6 p.m. and 6 a.m.). The rats were used for the experiment at a body weight of about 260 g. Such freely fed male HanWistar rats (n = 5 per group) were administered oral doses of vehicle or 1-[(3-cyano-pyridin-2-yl)methyl]-3-methyl-7-(2-butyn-1-yl)-8-[3-(R)-amino-piperidin-1-yl]-xanthine monohydrochloride at 0.1, 0.3, 1, and 3 mg/kg body weight. Blood samples were drawn from the retrobulbal venous plexus under isoflurane anaesthesia. Blood was collected in EDTA tubes prior to administration of vehicle or 1-[(3-cyano-pyridin-2-yl)methyl]-3-methyl-7-(2-butyn-1-yl)-8-[3-(R)-amino-piperidin-1-yl]-xanthine monohydrochloride and subsequently at 0.5, 1, 2, 3, 4, 7, and 24 hour time points post dose. Plasma was prepared following blood collection and frozen for determination of DPP-IV activity.

Overnight fasted male Beagle dogs (n = 4 per group) were administered oral doses of 1-[(3-cyano-pyridin-2-yl)methyl]-3-methyl-7-(2-butyn-1-yl)-8-[3-(R)-amino-piperidin-1-yl]-xanthine monohydrochloride at 3 and 12 mg/kg body weight. Blood samples were drawn from a butterfly inserted in either the right or left forearm vein. Blood was collected in EDTA tubes prior to administration of 1-[(3-cyano-pyridin-2-yl)methyl]-3-methyl-7-(2-butyn-1-yl)-8-[3-(R)-amino-piperidin-1-yl]-xanthine monohydrochloride and subsequently at 1, 2, 4, 8, and 24 hour time points post dose. Plasma was prepared following blood collection and frozen for determination of DPP-IV activity.

Overnight fasted Rhesus monkeys (n = 4; 2 males and 2 females) were administered oral doses of 1-[(3-cyano-pyridin-2-yl)methyl]-3-methyl-7-(2-butyn-1-yl)-8-[3-(R)-amino-piperidin-1-yl]-xanthine monohydrochloride at 1 mg/kg bw. Blood samples were drawn from a butterfly that was inserted into a forearm vein of restrained monkeys. Blood was collected in EDTA tubes prior to administration of 1-[(3-cyano-pyridin-2-yl)methyl]-3-methyl-7-(2-butyn-1-yl)-8-[3-(R)-amino-piperidin-1-yl]-xanthine monohydrochloride and subsequently at 0.5, 1, 2,4, 8, 24, 48, and 72 hour time points post dose. Plasma was prepared following blood collection and frozen for determination of DPP-IV activity.

In the rat, 1-[(3-cyano-pyridin-2-yl)methyl]-3-methyl-7-(2-butyn-1-yl)-8-[3-(R)-amino-piperidin-1-yl]-xanthine monohydrochloride (1 mg/kg) inhibited plasma DPP IV activity by 86% 7 hours post dose and by 64% 24 hours post dose. The ED50 (Dose to achieve 50% of maximal efficacy) for inhibition of plasma DPP IV activity in the rat has been determined as 0.4 mg/kg 7 h post dose and 0.6 mg/kg 24 h post dose.

In Beagle dogs and Rhesus monkeys, plasma DPP IV activity was inhibited 24 hours post dose by 76% (at 3 mg/kg), and by 54% (at 1 mg/kg), respectively.

Together, these data indicate a long duration of action for 1-[(3-cyano-pyridin-2-yl)methyl]-3-methyl-7-(2-butyn-1-yl)-8-[3-(R)-amino-piperidin-1-yl]-xanthine in different species.

### Example 3

**The effect of 1-[(3-cyano-pyridin-2-yl)methyl]-3-methyl-7-(2-butyn-1-yl)-8-[3-(R)-amino-piperidin-1-yl]-xanthine on glucose tolerance in normoglycaemic C57BI/6J mice**

The effect of 1-[(3-cyano-pyridin-2-yl)methyl]-3-methyl-7-(2-butyn-1-yl)-8-[3-(R)-amino-piperidin-1-yl]-xanthine on glucose tolerance in normoglycaemic C57BI/6J mice has been assessed according to the following protocol of an oral glucose tolerance test (OGTT).

Male C57BI/6J mice (C57BL/6J@Rj) were obtained from Janvier (France). Animals were fed ad libitum with a standard pelleted diet (Diet No. 3438 [containing 19 % protein, 4.5 % fat], Provimi Kliba, Switzerland) and had free access to water. Animals were individually housed with a 12 h/12 h light/dark cycle (lights out between 6 p.m. and 6 a.m.). The mice were used for the experiments at 9 weeks of age.

For the determination of the acute oral glucose tolerance test, a basal blood sample (pre-dose) was obtained in the morning from overnight fasted C57BI/6J mice (n = 7 per group) by cutting the tip of the tail without anaesthesia. Subsequently, the groups received a single oral administration of either vehicle or 1-[(3-cyano-pyridin-2-yl)methyl]-3-methyl-7-(2-butyn-1-yl)-8-[3-(R)-amino-piperidin-1-yl]-xanthine monohydrochloride at doses between 0.1 and 10 mg/kg bw, followed 45 min later by an oral administration of a glucose solution (2 g/kg bw, application volume 10 ml/kg bw). Additional blood samples were taken from the tip of the tail 30 min, 60 min, 90 min, 120 min, and 180 min after the glucose load, and blood glucose was measured. A final blood sample was drawn from the retrobulbal venous plexus under isoflurane anaesthesia 30 minutes after the last bleeding from the tail. EDTA plasma was prepared from this blood sample and frozen for determination of DPP-IV activity.

For the determination of the delayed oral glucose tolerance test, C57BI/6J mice (n = 5 per group) were orally administered with either vehicle or 1-[(3-cyano-pyridin-2-yl)methyl]-3-methyl-7-(2-butyn-1-yl)-8-[3-(R)-amino-piperidin-1-yl]-xanthine monohydrochloride at doses between 0.1 and 10 mg/kg bw in the afternoon before the oral glucose tolerance test. A basal blood sample (pre-load) was obtained the next morning after overnight fasting for 16 hours by cutting the tip of the tail without anaesthesia. Subsequently, the groups received an oral administration of a glucose solution (2 g/kg bw, application volume 10 ml/kg bw). Additional blood samples were taken from the tip of the tail 30 min, 60 min, 90 min, 120 min, and 180 min after the glucose load, and blood glucose was measured. A final blood sample was drawn from the retrobulbal venous plexus under isoflurane anaesthesia 30 minutes after the last bleeding from the tail. EDTA plasma was prepared from this blood sample and frozen for determination of DPP-IV activity.

The two-sided unpaired Student t-test was used for statistical comparison of the control group and the active groups with the 5 % level as the limit of statistical significance (*, P < 0.05; **, P < 0.01; ***, P < 0.001). The test was adjusted for unequal variances if the F-test for equal variances yielded a P-value < 0.05. Changes from baseline (pre-dose value) within each group were analyzed by a paired Student t-test.

The area under the curve (AUC) for the time course of blood glucose concentrations during an OGTT was calculated by the trapezoidal rule after correction for the baseline values.

Parameters for descriptive statistics, t-test and AUC were calculated using the program Microsoft Excel 2002 SP3.

ED50 values were calculated by nonlinear regression analysis with a Hill coefficient of 1. The values for the control group (0 mg/kg) were included into the calculation as a dose of 1xE -10 mg/kg. If regression analysis did not converge, a constraint (mean value of the control group) was imposed on the top of the curve.

For non-linear regression analysis, the program GraphPad Prism Version 4.03 was used.

The area under the blood-glucose-time-curves (AUC) for glucose excursion is suppressed by 38% after acute dosing of 1 mg/kg. When the compound is administered 16 hours before the oral glucose tolerance test (i.e. delayed setting), the same dose reduces the AUC for glucose excursion by 29%.

The ED50 for lowering the glucose AUC is 0.2 mg/kg in the acute setting, and 0.3 mg/kg in the delayed setting.

As a result it can be stated that 1-[(3-cyano-pyridin-2-yl)methyl]-3-methyl-7-(2-butyn-1-yl)-8-[3-(R)-amino-piperidin-1-yl]-xanthine improves the glucose tolerance in normoglycaemic C57BI/6J mice. These data additionally indicate a long duration of action for this compound.

### Example 4

**The effect of 1-[(3-cyano-pyridin-2-yl)methyl]-3-methyl-7-(2-butyn-1-yl)-8-[3-(R)-amino-piperidin-1-yl]-xanthine on glucose AUC in an oral glucose tolerance test (OGTT)**

A delayed oral glucose tolerance test (OGTT) has been applied to diabetic db/db mice and diabetic ZDF rats, as describes in the following.

Male obese diabetic *db*/*db* mice (C57BL/KSJ@Rj-db) were obtained from Janvier (France). Animals were fed ad libitum with a standard pelleted diet (Diet No. 3438 [containing 19 % protein, 4.5 % fat], Provimi Kliba, Switzerland) and had free access to water. Animals were housed in groups with a 12 h/12 h light/dark cycle (lights out between 6 p.m. and 6 a.m.). The mice used for these experiments were between 9 and 10 weeks of age.

Male Zucker Diabetic Fatty (ZDF) rats (ZDF/Crl-Leprfa) were obtained from Charles River (USA). Animals were fed ad libitum with a special pelleted diet (Diet No. 2437 [containing 4.5 % sucrose], Provimi Kliba, Switzerland) and had free access to water. Animals were housed in groups with a 12 h/12 h light/dark cycle (lights out between 6 p.m. and 6 a.m.). The rats were used for the experiments at 12 weeks of age.

The day before the OGTT, a blood sample from *db*/*db* mice or ZDF rats fasted for 2 hours was obtained by cutting the tip of the tail without anaesthesia and the animals were randomized for glucose (n = 7 per group). The animals were fed again for 2 hours and then orally administered with either vehicle or 1-[(3-cyano-pyridin-2-yl)methyl]-3-methyl-7-(2-butyn-1-yl)-8-[3-(R)-amino-piperidin-1-yl]-xanthine monohydrochloride at doses between 0.3 and 3 mg/kg bw. A basal blood sample (pre-load) was obtained the next morning after overnight fasting for 16 hours by cutting the tip of the tail without anaesthesia. Subsequently, the groups received an oral administration of a glucose solution (2 g/kg bw, application volume 10 ml/kg bw). Additional blood samples were taken from the tip of the tail 30 min, 60 min, 90 min, 120 min, and 180 min after the glucose load, and blood glucose was measured. A final blood sample was drawn from the retrobulbal venous plexus under isoflurane anaesthesia 30 minutes after the last bleeding from the tail. EDTA plasma was prepared from this blood sample and frozen for determination of DPP-IV activity.

In diabetic db/db mice, glucose AUC in this oral glucose tolerance test has been reduced by 39% when a 1 mg/kg dose had been administered 16 hours before the test. In diabetic ZDF rats, the same dose given 16 hours before the test suppressed glucose excursion by 35%.

These data show that the long duration of action of 1-[(3-cyano-pyridin-2-yl)methyl]-3-methyl-7-(2-butyn-1-yl)-8-[3-(R)-amino-piperidin-1-yl]-xanthine is also found in diabetic animals.

### Example 5

**The effect of 1-[(3-cyano-pyridin-2-yl)methyl]-3-methyl-7-(2-butyn-1-yl)-8-[3-(R)-amino-piperidin-1-yl]-xanthine on an oral glucose tolerance test (OGTT) in insulin-resistant fa/fa rats**

Male Zucker Fatty (*fa*/*fa*) rats (Ico-Zucker-fa/fa (Orl)) were obtained from Iffa (France). Animals were fed ad libitum with a standard pelleted diet (Diet No. 3438, Provimi Kliba, Switzerland) and had free access to water. Animals were housed in groups with a 12 h/12 h light/dark cycle (lights out between 6 p.m. and 6 a.m.). The rats were used for the experiments between 7 and 12 weeks of age.

*fa*/*fa* rats (n = 6 per group) were treated like the mice in example 3, delayed OGTT. 1-[(3-cyano-pyridin-2-yl)methyl]-3-methyl-7-(2-butyn-1-yl)-8-[3-(R)-amino-piperidin-1-yl]-xanthine has been dosed at 3 mg/kg 16 hours before the OGTT. The results of this experiment are summarized in table 1.

Male Zucker Fatty (fa/fa) rats (Ico-Zucker-fa/fa (Orl)) were obtained from Iffa (France). Animals were fed ad libitum with a standard pelleted diet (Diet No. 3438, Provimi Kliba, Switzerland) and had free access to water. Animals were housed in groups with a 12 h/12 h light/dark cycle (lights out between 6 p.m. and 6 a.m.). The rats were used for the experiments between 7 and 12 weeks of age.

GLP-1 was measured using a commercially available GLP-1 (active) ELISA Kit (Catalog# EGLP-35K, Linco Research Inc., St. Charles/MO, USA). Insulin was measured using a commercially available Rat Insulin ELISA Kit (Catalog# 90060, Crystal Chem Inc., Downers Grove/IL, USA).

**Table 1: Oral glucose tolerance test (OGTT) 16 h (=delayed setting) after 1-[(3-cyano-pyridin-2-yl)methyl]-3-methyl-7-(2-butyn-1-yl)-8-[3-(R)-amino-piperidin-1-yl]-xanthine monohydrochloride administration Parameter: Glucose; unit: mM**

| **Group 1:** | **Control** | | | | | | **AUC [mM * min]** |
|---|---|---|---|---|---|---|---|
| | **Preload** | **30 min** | **60 min** | **90 min** | **120 min** | **180 min** | **0-90 min** |
| **n** | 6 | 6 | 6 | 6 | 6 | 6 | 6 |
| **Mean** | 5.45 | 11.13 | 8.32 | 6.33 | 5.72 | 5.27 | 269.75 |
| **Median** | 5.35 | 10.80 | 7.85 | 6.50 | 5.80 | 5.40 | 253.50 |
| **SD** | 0.39 | 1.82 | 1.60 | 0.71 | 0.52 | 0.60 | 99.53 |
| **SEM** | 0.16 | 0.74 | 0.65 | 0.29 | 0.21 | 0.24 | 40.63 |
| **CV%** | 7.13 | 16.33 | 19.20 | 11.28 | 9.02 | 11.37 | 36.90 |
| **Change versus baseline** | | | | | | | |
| **Mean Diff.%** | | 104.3 | 52.6 | 16.2 | 4.9 | -3.4 | |
| **P** | | 0.0006 | 0.0091 | 0.0414 | 0.3772 | 0.5816 | |

| **Group 5: 1-[(3-cyano-pyridin-2-yl)methyl]-3-methyl-7-(2-butyn-1-yl)-8-[3-(R)-amino-piperidin-1-yl]-xanthine monohydrochloride (3 mg/kg)** | | | | | | | **AUC [mM** * **min]** |
|---|---|---|---|---|---|---|---|
| **n** | 5 | 5 | 5 | 5 | 5 | 5 | 5 |
| **Mean** | 4.60 | 7.90 | 6.40 | 6.20 | 6.04 | 5.96 | 177.00 |
| **Median** | 4.40 | 8.00 | 6.30 | 6.30 | 5.70 | 6.10 | 165.00 |
| **SD** | 0.46 | 1.29 | 0.53 | 0.45 | 0.48 | 0.34 | 56.57 |
| **SEM** | 0.21 | 0.58 | 0.24 | 0.20 | 0.21 | 0.15 | 25.30 |
| **CV%** | 10.08 | 16.38 | 8.27 | 7.21 | 7.91 | 5.76 | 31.96 |
| **Change versus baseline** | | | | | | | |
| **Mean Diff.%** | | 71.7 | 39.1 | 34.8 | 31.3 | 29.6 | |
| **P** | | 0.0047 | 0.0099 | 0.0004 | 0.0001 | 0.0017 | |
| **Change versus control** | | | | | | | |
| **Mean Diff.%** | -15.6 | -29.0 | -23.0 | -2.1 | 5.7 | 13.2 | -34.4 |
| **P** | 0.0090 | 0.0089 | 0.0312 | 0.7266 | 0.3124 | 0.0484 | 0.0055 |

As a result it was found that 1-[(3-cyano-pyridin-2-yl)methyl]-3-methyl-7-(2-butyn-1-yl)-8-[3-(R)-amino-piperidin-1-yl]-xanthine reduces the area under the glucose excursion curve by 34.4%. GLP-1 levels in the 1-[(3-cyano-pyridin-2-yl)methyl]-3-methyl-7-(2-butyn-1-yl)-8-[3-(R)-amino-piperidin-1-yl]-xanthine group were elevated vs. the control group at all time points and raised by 93% (vs. baseline), while the GLP-1 levels in the control group raised by only 63% after the glucose stimulus. The increase in GLP-1 levels after 1-[(3-cyano-pyridin-2-yl)methyl]-3-methyl-7-(2-butyn-1-yl)-8-[3-(R)-amino-piperidin-1-yl]-xanthine treatment was accompanied by further elevations in peak plasma insulin levels, compared to vehicle treatment.

Together, these data indicate that 1-[(3-cyano-pyridin-2-yl)methyl]-3-methyl-7-(2-butyn-1-yl)-8-[3-(R)-amino-piperidin-1-yl]-xanthine improves glucose control in disease-related rodent models.

### Example 6

### Inhibition of DPP-4 by 1-[(3-cyano-pyridin-2-yl)methyl]-3-methyl-7-(2-butyn-1-yl)-8-[3-(R)-amino-piperidin-1-yl]-xanthine in cat and rat plasma

Plasma obtained from 3 different cats and one Wistar rat as control were each incubated with a range of concentrations of 1-[(3-cyano-pyridin-2-yl)methyl]-3-methyl-7-(2-butyn-1-yl)-8-[3-(R)-amino-piperidin-1-yl]-xanthine monohydrochloride. Plasma DPP-4 activity was measured in he following way (which is also described in Eckhard et al. (2007), J. Med. Chem., Vol. 50, 6450-6453, Supporting Information).

Rat and cat plasma was used for ex vivo measurement of DDP-4 activity. Blood was collected in EDTA tubes and subjected to centrifugation. The resultant supernatant was aliquoted and frozen. Plasma DPP-4 activity was assayed after dilution (140-fold for rat plasma; 70-fold for mouse, dog, and monkey plasma) with assay buffer (100 mM Tris-HCI, 100 mM NaCl, pH 7.8; compound stock solutions in dimethylsulfoxide (DMSO), final DMSO concentration in the assay 1 %) prior to use.

The assay itself was initiated by mixing 50 µl of diluted plasma with 50 µl of diluted substrate (Ala-Pro- AFC) and performed in black flat-bottom 96-well plates. The plates were then incubated at room temperature for 1 h and fluorescence was measured at excitation/emission wavelengths of 405/535 nm. Data analysis was performed by calculating the fluorescence in the presence of the test compound compared to the fluorescence of the vehicle control after subtracting the background fluorescence.

From a plot of concentration against activity (equivalent to fluorescence units) kinetic data could be collected, e.g. the half-maximal inhibitory concentration (IC50) of the tested compound in the different animals, which data are given in the following table 2.

**Table 2: Inhibition of DPP-4 by 1-[(3-cyano-pyridin-2-yl)methyl]-3-methyl-7-(2-butyn-1-yl)-8-[3-(R)-amino-piperidin-1-yl]-xanthine monohydrochloride in cat and rat plasma**

| | **cat** 1 | **cat 2** | **cat 3** | **wistar rat** |
|---|---|---|---|---|
| **bottom** | 0.0 % | 0.0 % | 0.0 % | 0.0 % |
| **top** | 100.0 % | 100.0 % | 100.0 % | 100.0 % |
| **log IC 50** | - 8.712 | - 8.838 | - 8.736 | -8.710 |
| **hillslope** | - 0.7518 | -0.7102 | - 0.7615 | - 0.8462 |
| **IC 50** | 1.942e-009 M | 1.453e-009 M | 1.837e-009 M | 1.950e-009 M |

This in vitro study shows a high efficacy of 1-[(3-cyano-pyridin-2-yl)methyl]-3-methyl-7-(2-butyn-1-yl)-8-[3-(R)-amino-piperidin-1-yl]-xanthine for inhibiton of the plasma DPP-4 enzyme in cats.

### Example 7

### Exploratory single dose (0.3/1.0/3.0 mg/kg) PK/PD (GLP)

Several pharmacokinetic and pharmacodynamic (PK/PD) studies have been conducted in order to measure the effect of different doses of 1-[(3-cyano-pyridin-2-yl)methyl]-3-methyl-7-(2-butyn-1-yl)-8-[3-(R)-amino-piperidin-1-yl]-xanthine on DPP IV. These will be shown in the following examples.

The aim of this first study was to investigate the PK/PD of 1-[(3-cyano-pyridin-2-yl)methyl]-3-methyl-7-(2-butyn-1-yl)-8-[3-(R)-amino-piperidin-1-yl]-xanthine in the cat after a single oral administration of 0, 0.3, 1.0 and 3.0 mg/kg body weight. For this purpose, twelve male clinically healthy adult domestic short hair cats with a body weight range of 3.5 - 4.5 kg were used. All animals were treated once orally with placebo or the respective 1-[(3-cyano-pyridin-2-yl)methyl]-3-methyl-7-(2-butyn-1-yl)-8-[3-(R)-amino-piperidin-1-yl]-xanthine monohydrochloride dose formulation. Blood samples for the determination of this compound and DPP IV activity were collected at 0 (pre-dose), 0.5, 1, 2, 4, 8, 24, 36, 48, 72 and 96 hours after treatment and the activity of DPP IV has been determined like in example 6.

The resulting pharmacodynamic data from this experiment are summarized in following table 3; the value of 0 h has been set to 100 % for each treatment.

**Table 3: Exploratory single dose PK/PD (0.3/1.0/3.0 mg/kg); the data are given as DPP IV activity [in %] in cat plasma**

| **Time [h]** | **Placebo** | **0.3 mg/kg** | **1 mg/kg** | **3 mg/kg** |
|---|---|---|---|---|
| **0** | 100 | 100 | 100 | 100 |
| **0.5** | 97 | 15 | 11 | 16 |
| **1** | 103 | 13 | 11 | 13 |
| **2** | 94 | 13 | 12 | 12 |
| **4** | 94 | 15 | 12 | 14 |
| **8** | 97 | 19 | 15 | 17 |
| **24** | 80 | 54 | 42 | 49 |
| **36** | 102 | 84 | 67 | 66 |
| **48** | 101 | 93 | 80 | 85 |
| **72** | 102 | 95 | 91 | 101 |
| **96** | 100 | 95 | 92 | 95 |

As can be seen from these data, all doses of the compound relevant for the invention led to more than 80% inhibition of DPP IV.

### Example 8

### Exploratory single dose (0.01/0.1/1.0 mg/kg) PK/PD (GLP)

The investigation of the PK/PD values has been conducted in the same way as in example 7, with the variations described below.

The aim of this study was to investigate the PK/PD of 1-[(3-cyano-pyridin-2-yl)methyl]-3-methyl-7-(2-butyn-1-yl)-8-[3-(R)-amino-piperidin-1-yl]-xanthine monohydrochloride in the cat after a single oral administration of 0, 0.01, 0.1 and 1.0 mg/kg body weight of this compound (dosages were based on the free base).

Eleven clinically healthy male adult domestic short hair cats with a body weight range of 3.5 - 5.0 kg were used in this study. All animals were treated orally once via a stomach tube. Blood samples were drawn at 0 h (i.e. prior to treatment), 30 min as well as 1, 2, 4, 8, 12, 24, 36, 48, 72 and 96 h after treatment and the activity of DPP IV has been determined like in example 6.

The resulting pharmacodynamic data from this experiment are summarized in following table 4.

**Table 4: Exploratory single dose PK/PD (0.01/0.1/1.0 mg/kg); the data are given as DPP IV activity [in %] in cat plasma**

| **Time [h]** | **Placebo** | **0.01 mg/kg** | **0.1 mg/kg** | **1 mg/kg** |
|---|---|---|---|---|
| **0** | 100 | 100 | 100 | 100 |
| **0.5** | 99 | 73 | 23 | 10 |
| **1** | 91 | 66 | 17 | 10 |
| **2** | 85 | 71 | 16 | 8 |
| **4** | 84 | 77 | 24 | 10 |
| **8** | 86 | 85 | 39 | 12 |
| **12** | 95 | 88 | 50 | 15 |
| **24** | 98 | 90 | 67 | 27 |
| **36** | 94 | 87 | 83 | 63 |
| **48** | 92 | 88 | 82 | 72 |
| **72** | 91 | 85 | 91 | 80 |
| **96** | 87 | 90 | 90 | 86 |

As can be learnt from these data, the application of 1-[(3-cyano-pyridin-2-yl)methyl]-3-methyl-7-(2-butyn-1-yl)-8-[3-(R)-amino-piperidin-1-yl]-xanthine to cats at doses of 0.01, 0.1 and 1.0 mg/kg leads to a dose-related reduction/inhibition of the dipeptidyl peptidase IV (DPP IV) activity in plasma.

### Example 9

### Exploratory single dose (1.0/5.0/10.0 mg/kg) PK/PD (GLP)

The investigation of the PK/PD values has been conducted in the same way as in example 7, with the variations described below.

The aim of this study was to investigate the PK/PD behaviour of 1-[(3-cyano-pyridin-2-yl)methyl]-3-methyl-7-(2-butyn-1-yl)-8-[3-(R)-amino-piperidin-1-yl]-xanthine in the cat following a single oral administration of 0, 1, 5 and 10 mg/kg body weight (dosages were based on the free base: 1 mg 1-[(3-cyano-pyridin-2-yl)methyl]-3-methyl-7-(2-butyn-1-yl)-8-[3-(R)-amino-piperidin-1-yl]-xanthine monohydrochloride corresponds to 0.923 mg 1-[(3-cyano-pyridin-2-yl)methyl]-3-methyl-7-(2-butyn-1-yl)-8-[3-(R)-amino-piperidin-1-yl]-xanthine free base).

Twelve clinically healthy male adult domestic short hair cats with a body weight range of 3.5 - 4.7 kg were used in this study. All animals were treated orally once via a stomach tube. Blood samples for pharmacokinetic and pharmacodynamic (DPP IV activity) investigations were drawn at 0 h (i.e. prior to treatment), 30 min as well as 1, 2, 4, 8, 8.5, 9, 12, 24, 36, 48, 72, 84 and 96 h after treatment. Blood samples for pharmacodynamic (glucagon-like peptide 1 concentrations) investigations were drawn at -16 (i.e. prior to feeding), -15.5, -15 h as well as at 8 h (i.e. prior to feeding), 8.5 and 9 h after treatment.

The results of this experiment are summarized in following table 5.

**Table 5: Exploratory single dose PK/PD (1.0/5.0/10.0 mg/kg); the data are given as DPP IV activity [in %] in cat plasma**

| **Time [h]** | **Placebo** | **1 mg/kg** | **5 mg/kg** | **10 mg/kg** |
|---|---|---|---|---|
| **0** | 100 | 100 | 100 | 100 |
| **0.5** | 86 | 8.0 | 7.7 | 8,0 |
| **1** | 91,7 | 7,0 | 7,0 | 7,3 |
| **2** | 93,3 | 7,0 | 7,0 | 7,3 |
| **4** | 86,3 | 7,3 | 7,7 | 9,0 |
| **8** | 86,0 | 9,3 | 8,3 | 9,0 |
| **8.5** | 93,0 | 9,7 | 8,7 | 9,7 |
| **9** | 93,3 | 9,7 | 9,0 | 9,3 |
| **12** | 93,7 | 12,3 | 9,3 | 9,7 |
| **24** | 86,3 | 22,3 | 14,7 | 12,3 |
| **36** | 90,3 | 43,0 | 25,7 | 16,3 |
| **48** | 89,3 | 59,0 | 42,7 | 20,0 |
| **72** | 89,3 | 74,7 | 72,3 | 37,0 |
| **84** | 93,0 | 84,3 | 90,0 | 58,3 |
| **96** | 91,7 | 81,7 | 87,7 | 67,3 |

No specific clinical signs were observed after treatment with the test or control article. With respect to PD, minimum mean DPP IV values of 7 % were noted in all three groups. Values remained ≤ 20 % in groups treated with 1.0 mg/kg, 5.0 mg/kg and 10.0 mg/kg up to 12, 24 and 48 h, respectively. In the groups treated with 1-[(3-cyano-pyridin-2-yl)methyl]-3-methyl-7-(2-butyn-1-yl)-8-[3-(R)-amino-piperidin-1-yl]-xanthine, an increase of the mean GLP-1 concentration was observed after feeding.

PK data for 1-[(3-cyano-pyridin-2-yl)methyl]-3-methyl-7-(2-butyn-1-yl)-8-[3-(R)-amino-piperidin-1-yl]-xanthine free base were determined with a validated LC-MS/MS method and are summarized here in in table 6.

**Table 6: Pharmacokinetic data: Exploratory single dose PK/PD (1.0/5.0/10.0 mg/kg)**

| **Parameter** | | **1.0 mg/kg** | **5.0 mg/kg** | **10 mg/kg** |
|---|---|---|---|---|
| **tmax [hour]** | mean | 1.333 | 1.833 | 1.333 |
| **Cmax [nmol/L]** | mean | 174.5667 | 931.3333 | 5470.0000 |
| **AUC_{0→∞} [nmol·h/I]** | mean | 1 144.4828 | 5476.4590 | 26273.0654 |
| **T_{1/2} [hour]** | mean | 25.408 | 28.762 | 37.560 |
| **MRT [hour]** | mean | 12.312 | 6.761 | 5.734 |

These data demonstrate, that the application of 1-[(3-cyano-pyridin-2-yl)methyl]-3-methyl-7-(2-butyn-1-yl)-8-[3-(R)-amino-piperidin-1-yl]-xanthine to cats at doses of 1.0, 5.0 and 10.0 mg/kg leads to a dose-related reduction/inhibition of the dipeptidyl peptidase IV (DPP IV) activity in plasma and a dose relation in the exposure (Cₘₐₓ). 1-[(3-cyano-pyridin-2-yl)methyl]-3-methyl-7-(2-butyn-1-yl)-8-[3-(R)-amino-piperidin-1-yl]-xanthine has a long half live of over 25 hours in this study.

The The PK-PD correlation plotted on a graph (x-axis: plasma concentration; y-axis: % inhibition) demonstrates an sigmoidal slope. Maximum DPP-4 inhibition was reached at 15-30 nmol/I 1-[(3-cyano-pyridin-2-yl)methyl]-3-methyl-7-(2-butyn-1-yl)-8-[3-(R)-amino-piperidin-1-yl]-xanthine CL.There was no PK/PD correlation for concentrations above this threshold. DPP-4 activity correlates with actual concentration, not with AUC or exposure.

### Example 10

### Exploratory multiple dose PK/PD (1.0/3.0/9.0 mg/kg) (GLP)

The aim of this study was to investigate the PK/PD effects of 1-[(3-cyano-pyridin-2-yl)methyl]-3-methyl-7-(2-butyn-1-yl)-8-[3-(R)-amino-piperidin-1-yl]-xanthine in cats following repeated oral administration of 0, 1, 3 and 9 mg/animal over 14 days corresponding to a dose of 0, 0.25, 0.75 and 2.25 mg/kg body weight for a cat weighing 4 kg. (Dosages were based on the free base: 1 mg 1-[(3-cyano-pyridin-2-yl)methyl]-3-methyl-7-(2-butyn-1-yl)-8-[3-(R)-amino-piperidin-1-yl]-xanthine monohydrochloride corresponds to 0.923 mg 1-[(3-cyano-pyridin-2-yl)methyl]-3-methyl-7-(2-butyn-1-yl)-8-[3-(R)-amino-piperidin-1-yl]-xanthine free base).

For this purpose, twelve clinically healthy male adult domestic short hair cats with a body weight range of 3.5 - 4.8 kg were used. The animals were randomly allocated to 4 groups, 3 animals per group. All animals were treated once daily orally for 14 days (i.e. on days 0-13). The test article was administered via capsules at doses of 1 (group II), 3 (group III) or 9 (group IV) mg of the monohydrochloride compound per animal; group I received placebo serving as controls. Blood samples for PK and PD (DPP IV activity) investigations were drawn at 0 h (i.e. prior to the 1st treatment), 0.5, 1, 2, 4, 8, 12 and 24 h after the 1st treatment as well as 24 h after the 3rd, 5th, 7th, 9th, 11th and 13th treatment and 0.5, 1, 2, 4, 8, 12 and 24 h after the 14th treatment. No clinical signs specifically attributable to the test article were noted throughout the study period.

With respect to PD (see table 6), after the treatment on day 0 minimum mean DPP IV values of 9, 8 and 7 % were noted in the respective groups at 1 h (group II), 2 and 4 h (group III) and 1 and 2 h (group IV). Mean values remained ≤ 20 % in groups II, III and IV up to 8, 12 and 24 h, respectively. After treatment on days 2 - 12 (animals were treated daily with the test article, blood samples were collected 24 h after the 3rd, 5th, 7th, 9th, 11th and 13th treatment at 72, 120, 168, 216, 264 and 312 h), mean DPP IV activity of groups II, III and IV varied to some extent with ranges of 33 - 63 %, 19 - 41 % and 14 - 18 %, respectively, revealing a clear dose-relationship. After treatment of groups II, III and IV on day 13, mean DPP IV activities again decreased quickly to 14, 9 and 10 % already at 0.5 h after the 14th treatment (i.e. at 312.5 h). Mean values remained ≤ 20 % in groups II, III and IV up to 4, 12 and 24 h after the 14th treatment (i.e. 316, 324 and 336 h), respectively.

The resulting pharmacodynamic data from this experiment are summarized in following table 7.

**Table 7: Exploratory multiple dose PK/PD (1.0/3.0/9.0 mg/kg) over 14 days; the data are given as DPP IV activity [in %] in cat plasma**

| **Time [h]** | **Placebo** | 1 **mg/kg** | 3 **mg/kg** | 9 **mg/kg** |
|---|---|---|---|---|
| **0** | 100 | 100 | 100 | 100 |
| **0.5** | 84,3 | 31,3 | 16,0 | 8,0 |
| **1** | 94,7 | 8,7 | 10,3 | 7,3 |
| **2** | 93,0 | 9,7 | 8,0 | 7,0 |
| **4** | 91,3 | 11,7 | 8,0 | 8,0 |
| **8** | 96,3 | 17,0 | 11,3 | 9,3 |
| **12** | 99,0 | 28,0 | 14,0 | 10,7 |
| **24** | 96,7 | 57,7 | 31,7 | 18,0 |
| **72** | 90,0 | 35,3 | 23,3 | 14,3 |
| **120** | 87,3 | 63,0 | 31,7 | 17,7 |
| **168** | 90,0 | 54,0 | 41,0 | 14,7 |
| **216** | 90,7 | 57,0 | 31,7 | 15,0 |
| **264** | 26,0 | 32,7 | 19,3 | 14,0 |
| **312** | 84,3 | 61,3 | 34,0 | 17,3 |
| **312,5** | 88,3 | 13,7 | 8,7 | 10,0 |
| **313** | 92,3 | 14,0 | 8,3 | 7,7 |
| **314** | 87,7 | 13,3 | 8,7 | 7,7 |
| **316** | 84,0 | 14,7 | 9,7 | 8,0 |
| **320** | 86,7 | 22,3 | 12,3 | 10,3 |
| **324** | 81,7 | 32,3 | 15,0 | 12,0 |
| **336** | 83,0 | 67,7 | 31,3 | 18,7 |

Mean DPP-4 activity of the group which received 1 mg/cat ranged from 33-63, from 19-41 in the group which received 3 mg/cat and from 14-18% in the group which received 9 mg/cat, revealing a clear dose-relationship. No clinical signs specifically attributable to the test article were noted throughout the study period (14 days).

### Example 11

**Oral glucose tolerance test (OGTT) in obese/non-obese cats (Non-GLP) without compound 1-[(3-cyano-pyridin-2-yl)methyl]-3-methyl-7-(2-butyn-1-yl)-8-[3-(R)-amino-piperidin-1-yl]-xanthine**

Oral glucose tolerance testing according to example 4 was performed in 10 obese and 9 lean age-matched neutered cats in the following way.

Cats were fasted overnight and then lightly tranquilized with tiletamine/zolazepam (2 mg per cat intravenously (Fort Dodge Animal Health, Ft. Dodge, IA, USA). Glucose was administered via gastric tube (2 g/kg body weight; 80% w/v) after a baseline blood sample had been obtained. Post-glucose blood samples were collected at 30 min and at 1, 2, 3, 4, and 5 h for measurements of glucose, insulin, and active GLP-1.

Glucose measurements were performed using a colorimetric glucose oxidase method (DCL, Oxford, CT, USA). Plasma insulin was measured as described in Hoenig et al. (1989), J. Endocrinol., vol. 121, p. 249-251. The concentrations of active GLP-1 were measured with an ELISA kit from Linco (St. Charles, MO, USA). The assay has been validated for use in cats in our laboratory. All samples were tested in duplicate. The standard curve for serial dilutions of serum from clinically normal lean and obese cats was observed to be parallel to the standard curve for active GLP-1 standards. Addition of 3 concentrations of GLP-1 standard to feline serum resulted in mean±SD recovery of 87.5±9.0%. The assay had a working range of 2-100 pM. The interassay coefficient of variation (CV) was 7.6%, and the intra-assay CV was 12.1 %.

The area under the curve (AUC) was estimated by the sum of all the trapezoids and triangles bounded by the time versus concentration curve and was mathematically calculated as the integral of the curve above 0 concentration. Area under the curve data were checked for normality and concentrations were checked for multinormality (Systat 12, Systat Inc., Richmond, CA). Area under the curve data did not have a normal distribution for any parameter, and they were not transformable to normal. Glucose values from 0 to 240 min had a multinormal distribution. Log-transformed values for insulin and GLP-1 had a multinormal distribution from both 0 to 240 min and 0 to 300 min. Area under the curve data were analyzed using Monte Carlo sampling, implemented using Resampling Stats for Excel 2007 (Statistics.com LLC, Arlington, VA, USA).

As a result, all cats responded to oral administration of 2 g/kg glucose with a prompt increase in glucose, insulin, and GLP-1, measured according to the instruction of the ELISA kit from Linco Research (St. Charles, MO, USA). This assay was previously validated for cat plasma. There was a significant difference between lean and obese cats in the area under the curve for glucose, insulin, and GLP-1. The AUC for glucose and insulin was significantly greater in obese cats than lean cats, whereas the AUC for GLP-1 was significantly lower in obese cats. The results of this study show that cats are capable to respond to an oral glucose challenge with a robust increase in glucose with peak glucose concentrations.

### Example 12

**Oral glucose tolerance test (OGTT) in normal cats more than 1 h after oral administration of 9 mg 1-[(3-cyano-pyridin-2-yl)methyl]-3-methyl-7-(2-butyn-1-yl)-8-[3-(R)-amino-piperidin-1-yl]-xanthine per cat monohydrochloride (Non-GLP)**

An OGTT like in example 11 was performed in 6 male and 6 female animals beginning 1 h after oral administration of 9 mg 1-[(3-cyano-pyridin-2-yl)methyl]-3-methyl-7-(2-butyn-1-yl)-8-[3-(R)-amino-piperidin-1-yl]-xanthine monohydrochloride or Placebo (2 groups with 6 animals each; 3 male and 3 females per group).

The result is summarized in following table 8.

**Table 8: GLP-1 concentrations [pmol/I] over time during an OGTT study in normal cats more than 1 h after oral administration of 9 mg 1-[(3-cyano-pyridin-2-yl)methyl]-3-methyl-7-(2-butyn-1-yl)-8-[3-(R)-amino-piperidin-1-yl]-xanthine monohydrochloride or Placebo per cat (Non-GLP); the results are given as concentration of GLP-1 in pmol/l.**

| **Time after drug administration [min]** | **Placebo** | 9 **mg/cat** |
|---|---|---|
| **60** | 3,90 | 3,70 |
| **65** | 3,90 | 3,92 |
| **70** | 4,58 | 4,54 |
| **75** | 5,99 | 7,28 |
| **90** | 7,76 | 16,31 |
| **120** | 8,20 | 26,93 |
| **180** | 7,95 | 23,25 |
| **240** | 7,33 | 14,45 |
| **300** | 6,50 | 9,60 |
| **360** | 5,23 | 6,04 |

In this study the DPP IV activity was inhibited more than 80 %.

These data demonstrate that the GLP-1 increase in the 1-[(3-cyano-pyridin-2-yl)methyl]-3-methyl-7-(2-butyn-1-yl)-8-[3-(R)-amino-piperidin-1-yl]-xanthine group is significantly higher compared to Placebo and thus due to the inhibition of the DPP IV activity.

## Claims

1. Pharmaceutical composition comprising 1-[(3-cyano-pyridin-2-yl)methyl]-3-methyl-7-(2-butyn-1-yl)-8-[3-(R)-amino-piperidin-1-yl]-xanthine or a pharmaceutically acceptable salt thereof for the treatment of a metabolic disorder or metabolic disease of a predominantly carnivorous non-human animal.

2. Pharmaceutical composition according to claim 1, comprising a pharmaceutically acceptable salt of 1-[(3-cyano-pyridin-2-yl)methyl]-3-methyl-7-(2-butyn-1-yl)-8-[3-(R)-amino-piperidin-1-yl]-xanthine preferably 1-[(3-cyano-pyridin-2-yl)methyl]-3-methyl-7-(2-butyn-1-yl)-8-[3-(R)-amino-piperidin-1-yl]-xanthine monohydrochloride.

3. Pharmaceutical composition according to claim 1 or 2, where the predominantly carnivorous non-human animal is a predominantly carnivorous mammal, more preferred a dog (canine) or a cat (feline), even more preferred a cat (feline).

4. Pharmaceutical composition according to one of claims 1 to 3, where the metabolic disorder or metabolic disease is ketoacidosis, pre-diabetes, diabetes mellitus type 1 or type 2, insulin resistance, obesity, hyperglycemia, hyperinsulinemia, elevated blood levels of fatty acids, hyperlipidemia and/or elevated blood levels of glycerol, Syndrome X, atherosclerosis, inflammation of the pancreas and/or inflammation of adipose tissue, preferably ketoacidosis, pre-diabetes and/or diabetes mellitus type 1 or type 2, more preferred diabetes mellitus type 2.

5. Pharmaceutical composition according to one of claims 1 to 4, comprising pharmaceutically acceptable excipients, preferably a carrier or vehicle for the pharmaceutically active compound.

6. Pharmaceutical composition according to claim 5, comprising pharmaceutically acceptable excipients which ameliorate the chewability and/or the palatability of the composition, especially the chewability and/or the palatability for a dog and/or a cat, more preferred the chewability and/or the palatability of the composition for a cat.

7. Pharmaceutical composition according to one of claims 1 to 6 for oral or parenteral administration, preferably for oral administration.

8. Pharmaceutical composition according to one of claims 1 to 7 for the administration of 1 to 5 doses per day, preferably 1 or 2 doses per day, more preferred 1 dose per day.

9. Pharmaceutical composition according to one of claims 1 to 8 for the administration of 0.1 to 100 mg of the active ingredient per kg per day, preferably of 0.5 to 50 mg/kg per day, more preferred of 0.75 to 25 mg/kg per day, even more preferred of 1.0 to 15 mg/kg per day.

10. Pharmaceutical composition according to one of claims 1 to 9 for reaching a maximum blood plasma concentration of the active ingredient within the range of 6 to 10 nmol per I.

11. Pharmaceutical composition according to one of claims 1 to 10 which is a solid formulation.

12. Pharmaceutical composition according to claim 11, **characterized in that** the weight of the whole solid formulation of one dose is in the range of 50 to 3000 mg, preferred 75 to 1000 mg, more preferred 80 to 500 mg and most preferred 90 to 250 mg.

13. Pharmaceutical composition according to one of claims 1 to 12 comprising another pharmaceutically effective compound which is effective against a metabolic disorder or metabolic disease, preferably against ketoacidosis, pre-diabetes, diabetes mellitus type 1 or type 2, insulin resistance, obesity, hyperglycemia, hyperinsulinemia, elevated blood levels of fatty acids, hyperlipidemia and/or elevated blood levels of glycerol, Syndrome X, atherosclerosis, inflammation of the pancreas and/or inflammation of adipose tissue, more prefered against ketoacidosis, pre-diabetes and/or diabetes mellitus type 1 or type 2, even more preferred against diabetes mellitus type 2.

14. Pharmaceutical composition according to claim 13 comprising one or more compounds selected from SGLT-2 inhibitors, preferably selected from the group consisting of:
(1) Dapagliflozin;
(2) Remogliflozin or Remogliflozin etabonate;
(3) Sergliflozin or Sergliflozin etabonate;
(4) 1-Chloro-4-(ß-D-glucopyranos-1-yl)-2-(4-ethyl-benzyl)-benzene;
(5) (1S)-1,5-Anhydro-1-[5-(azulen-2-ylmethyl)-2-hydroxyphenyl]-D-glucitol;
(6) (1S)-1,5-Anhydro-1-[3-(1-benzothien-2-ylmethyl)-4-fluorophenyl]-D-glucitol;
(7) Thiophen derivative of the formula (7-1) 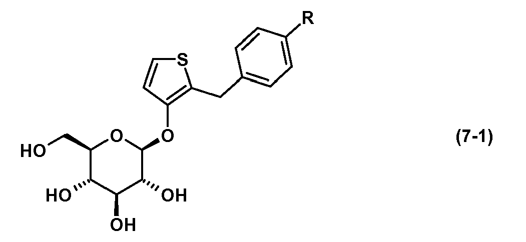 wherein R denotes methoxy or trifluoromethoxy;
(8) 1-(ß-D-glucopyranosyl)-4-methyl-3-[5-(4-fluorophenyl)-2-thienylmethyl]benzene;
(9) Spiroketal derivative of the formula (9-1): 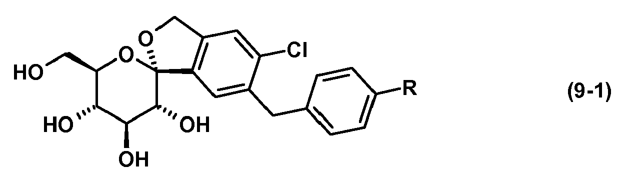 wherein R denotes methoxy, trifluoromethoxy, ethoxy, ethyl, isopropyl or tert. butyl;
(10) a glucopyranosyl-substituted benzene derivative of the formula (10-1) 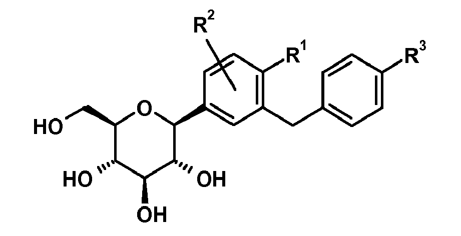 wherein R¹ denotes Cl, methyl or cyano; R² denotes H, methyl, methoxy or hydroxy and R³ denotes ethyl, cyclopropyl, ethinyl, ethoxy, (*R*)-tetrahydrofuran-3-yloxy or (*S*)-tetrahydrofuran-3-yloxy,
(11) a pyrazole-O-glucoside derivative of the formula (11-1) 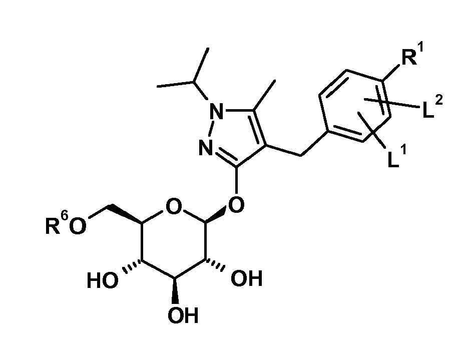 wherein
R¹ denotes C₁₋₃-alkoxy,
L¹, L² independently of each other denote H or F,
R⁶ denotes H, (C₁₋₃-alkyl)carbonyl, (C₁₋₆-alkyl)oxycarbonyl, phenyloxycarbonyl, benzyloxycarbonyl or benzylcarbonyl,
or a pharmaceutically acceptable salt, hydrate or solvate thereof.

15. Pharmaceutical composition according to one of claims 1 to 14, designed for a co-administration with insulin, preferably designed for a simultaneous, a sequential and/or a chronologically staggered coadministration with insulin.

16. Use of a pharmaceutical composition according to one of claims 1 to 15 for the treatment of a metabolic disorder or metabolic disease of a predominantly carnivorous non-human animal.

17. Use of 1-[(3-cyano-pyridin-2-yl)methyl]-3-methyl-7-(2-butyn-1-yl)-8-[3-(R)-amino-piperidin-1-yl]-xanthine or a pharmaceutically acceptable salt thereof for the preparation of a pharmaceutical composition for the treatment of a metabolic disorder or metabolic disease of a predominantly carnivorous non-human animal.

18. Use according to claim 17 comprising one or more additional compounds selected from SGLT-2 inhibitors, preferably selected from the group defined in claim 14.

19. Use of 1-[(3-cyano-pyridin-2-yl)methyl]-3-methyl-7-(2-butyn-1-yl)-8-[3-(R)-amino-piperidin-1-yl]-xanthine or a pharmaceutically acceptable salt thereof for the treatment of a metabolic disorder or metabolic disease of a predominantly carnivorous non-human animal.

20. Use according to claim 19 comprising one or more compounds selected from SGLT-2 inhibitors preferably selected from the group defined in claim 14.
